# EUROPEAN PATENT APPLICATION

(11) **EP 1 627 915 A1**
(43) Date of publication of application: **22.02.2006**
(21) Application number: 04730670.9
(22) Date of filing: 30.04.2004
(51) Int. Cl.: C12N 15/00, C12Q 1/68, A01H 1/00, A01K 67/00, A61D 19/00

(54) **ARRAY HAVING SUBSTANCES FIXED ON SUPPORT ARRANGED WITH CHROMOSOMAL ORDER OR SEQUENCE POSITION INFORMATION ADDED THERETO, PROCESS FOR PRODUCING THE SAME, ANALYTICAL SYSTEM USING THE ARRAY AND USE OF THESE**

(30) Priority: 01.05.2003 JP 2003126667; 26.03.2004 JP 2004093826; 26.03.2004 JP 2004093824; 26.03.2004 JP 2004093825
(71) Applicant: Japan Science and Technology Agency, Kawaguchi-shi, Saitama 332-0012 (JP)
(72) Inventor: TAKEDA, Kazuyoshi, Kurashiki-shi, Okayama 710-0031 (JP); SATO, Kazuhiro, Kurashiki-shi, Okayama 710-0031 (JP)
(74) Representative: Bentz, Jean-Paul
(86) International application number: PCT/JP2004/006284
(87) International publication number: WO 2004/097015

(57) **Abstract**

In fabricating various types of arrays such as a micro array, different kinds of biosubstances, or synthetic substances interacting with the biosubstances, are arranged and immobilized on a support such that the chromosomal order of base sequence blocks, corresponding to the biosubstances, is ascertainable. The biosubstances may be nucleic acids such as DNA, or polypeptides such as protein. The synthetic substances may be compounds that react with the biosubstances. By thus specifying the order of the biosubstances or synthetic substances immobilized on the support, the array can be used, for example, for screening in variety improvement of living organisms.

## Description

### TECHNICAL FIELD

The present invention relates, for example, to a novel array and a fabrication method of the array, various analytical systems using the array, and representative methods of using these techniques.

More specifically, the invention relates to (1) an array, such as a DNA micro array, in which biosubstances derived from a living organism, or synthetic substances that interact with the biosubstances, are immobilized on a support by being arranged in an orderly manner, (2) a system for analyzing a genotype of the organism of interest for display, and, in particular a genotype analyzing and display system that enables locations of crossovers on the chromosomes to be visually recognized in hybrid individuals obtained by crossing, (3) a system for analyzing quantitative trait loci of the organism of interest, and representative methods of using it, and in particular a quantitative loci analyzing system for analyzing QTL by effectively using the analysis result obtained from a nucleic acid array, and (4) a gene interaction analyzing system, and in particular a gene interaction analyzing system for effectively analyzing which genes or a group of genes are associated with the traits or genes being analyzed, by effectively using the analysis result obtained from a nucleic acid array. The invention also relates to representative methods of using such arrays and analyzing systems.

### BACKGROUND ART

With the recent advance of the worldwide genome project, the entire genomes of many model organisms have been sequenced. Sequencing of the entire genomes of many other organisms are underway as in the sequencing of the human genome in the Human Genome Project. As evidenced by these advances, research in molecular biology has entered the post-genome (post-sequence) era.

In the post-genome era, a new approach has been used for the analysis of genome functions. Specifically, the emphasis of genome function analysis has shifted, rather drastically, from the conventional pinpoint approach whereby analysis is made by cloning individual genes associated with certain living phenomena, to a systematic and comprehensive approach whereby gene functions are analyzed on a genome scale.

The genome information is also used for the analysis of transcripts and proteins. Specifically, transcriptome analysis and proteome analysis have won the recognition. The transcriptome analysis is used for the analysis of transcripts, whereby the expression of all transcripts in an organism or cells are analyzed both systematically and comprehensively using genome information. The proteome analysis is a systematic and comprehensive method of analyzing proteins, in which the properties or expression of all proteins expressed at any given location and any given time in an organism or cells are analyzed using genome information.

For the systematic and comprehensive analyses, various array techniques are often used. The array technique refers to a technique using an array, in which biosubstances, such as DNA or various proteins obtained from the organism of interest being analyzed, or synthetic substances (for example, compounds with hydrophobic groups or ion exchange groups) that interact with such biosubstances are immobilized on a support in an orderly manner.

With the array technique, the systematic and comprehensive analysis can be performed efficiently. For example, for the analysis of gene transcription control mechanism, it is required to measure transcription level of genes, which varies according to the state of the cell. For this purpose, use of a DNA micro array, one form of the array technique, allows for systematic measurement of transcription level of several thousand to several ten thousand of genes (see Non-Patent Documents 1-6, for example).

Among such DNA micro array techniques, one that has been widely used is the DNA micro array technique developed by Affymetrix. In this technique, oligonucleotides are directly synthesized on a silica substrate using a microfabrication technique employed in the fabrication of semiconductors (see Patent Document 1, for example).

For example, for the analysis of gene transcription control mechanism, it is required to measure transcription level of genes, which varies according to the state of the cell. For this purpose, use of a DNA micro array, one form of the array technique, allows for systematic measurement of transcription level of several thousand to several ten thousand of genes. Thus, through hybridization, the nucleic acid array such as the DNA micro array can produce a large amount of data concerning gene expression.

However, it is practically impossible to manually process the gene expression data obtained from the nucleic acid array since the amount of data obtained in biotechnology is enormous. In view of this, there have been proposed various types of bioinformatics techniques, whereby a large volume of data is analyzed using computers. As a technique of analyzing gene expression data, it has been known to analyze gene expression patterns in clusters, as disclosed in Patent Document 2, or analyze gene expression data based on parameters and use it for clinical purposes, as disclosed in Patent Document 3.

With the large data volume to be analyzed, the analysis may yield complex results. Therefore, the bioinformatics technique requires a technique of desirably displaying the analysis results. For example, as a technique concerning gene expression display, a technique for two-dimensionally displaying expression level has been known, as disclosed in Patent Document 4.

With the recent advance in the gene modification technique, alien genes have been introduced into various plants to confer new traits. Actual application of such plants as crop plants is also underway. The development of genetically modified crops (GMO) was once believed to have a promising future in bio-industries. However, the GMO could not win customer acceptance, and, today, safety of processed foods is often promoted by not using GMO.

It is therefore inconceivable that the traditional crossing or mutant induction will fade away in the variety improvement of crops. On the contrary, for improving the market value of crops or processed foods using crops, crossing or other traditional methods are still favored as a primary method of variety improvement of crops.

However, in actual variety improvement by crossing for example, a group of hybrid individuals, numbering several thousand to several tens of thousand, is screened for useful individuals by observing or analyzing traits of the hybrid individuals. As such, the efficiency of screening for superior individuals is considerably poor.

The array technique and bioinformatics technique are believed to facilitate the variety improvement employing traditional crossing.

One known technique of crossing is screening of a genotype using genetic markers. In variety improvement using genetic markers, it is important to recognize loci associated with target quantitative traits (QTL). The quantitative traits are governed by the polygene system, and therefore it is not possible to directly deal with the effects of expression of individual genes. This is where statistical analysis is important for the recognition of QTL. Specifically, in order to recognize QTL, selected genetic markers are scattered along the entire chromosomes, and any linkage between the genetic markers and the quantitative traits is determined in order to map locations of QTL on a linkage map.

The QTL analysis requires development of genetic markers or other materials such as hybrid lines (family lines), which are used to construct a linkage map. In addition, the QTL analysis produces a vast amount of information concerning analysis, such as measurement of traits, or typing of genetic markers (number of genetic markers 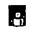 number of individuals). The array technique and bioinformatics technique are considered to facilitate the QTL analysis.

In the analysis of gene expression data, the term "expression profile" is used to refer to patterns of gene expression or amount which vary depending on the cell type or cell stage. By measuring and analyzing the expression profile, important findings concerning gene functions or regulation mechanisms can be obtained. Such findings can be effectively used for the variety improvement of industrially useful species. In the case of humans, the analysis of expression profile can yield useful results for drug discovery, pharmacology, toxicology, and diagnosis.

One technique of expression profile analysis is one that employs clustering, as disclosed in Patent Document 2 and Patent Document 5. In clustering, a group of genes that shows similar expression patterns under different measurement conditions is identified and sorted into clusters on a nucleic acid array. Another technique is one that analyzes expression networks between genes, as disclosed in Patent Document 6. The expression level of a gene is directly or indirectly regulated by other genes, and therefore finding expression networks between genes provides important information in the expression profile analysis, as does clustering.

For human applications, Patent Document 7 discloses an evaluation index estimation technique, in which genes for quantitatively estimating an evaluation index of interest are suitably selected from data obtained from each sample. For example, in the score measuring changes in gene expression profile caused by human illness, the number of samples is considerably smaller than the number of genes on which changes in expression level are measured, owning to the difficulty in collecting a large number of samples. Thus, it is often difficult to analyze the correlation with the illness by a common statistical method. In order to overcome such problems, the technique disclosed in Patent Document 7 extracts genes closely related to an evaluation index of interest and estimates evaluation index data.
[Non-Patent Document 1]
   *Genome Functions, Expression Profile and Transcriptome;* Editor-in-Chief, Ken-ich Matsubara, Yoshiyuki Sakaki, Nakayama-Shoten Co., Ltd., published on September 13, 2000
[Non-Patent Document 2]
   *DNA Micro Array*; chief translator, Ikunoshinn Kato, Maruzen, published on September 25, 2000
[Non-Patent Document 3]
   *DNA Micro Array Practical Manual for Successful Data Acquisition, Basic Principle, from Chip Fabrication to Bioinformatics*, Editor-in-Chief, Yoshihide Hayashizaki, YODOSHA Co., Ltd., published on December 1, 2000
[Non-Patent Document 4]
   *Concise and Practical Introductions to DNA Micro Array Data Analysis,* YODOSHA Co., Ltd., published on November 20, 2002
[Non-Patent Document 5]
   *DNA Microarrays* Associate Editor: Kaaren Janssen, Cold Spring Harbor Laboratory Press, 2003
[Non-Patent Document 6]
   *Microarray Analysis,* Mark Schena, John Wiley & Sons, Inc., 2003
[Patent Document 1]
   Japanese Unexamined Patent Publication No. 228999/2000 (*Tokukai* 2000-228999; published on August 22, 2000)
[Patent Document 2]
   Japanese Unexamined Patent Publication No. 342299/2000 (*Tokukai* 2000-342299; published on December 12, 2000)
[Patent Document 3]
   Japanese PCT Laid-Open Publication No. 508853/2003 (published on March 4, 2003; International Publication No. WO01/016860, published on March 8, 2001)
[Patent Document 4]
   Japanese Unexamined Patent Publication No. 342000/1999 (Tokukaihei 11-342000; published on December 14, 1999)
[Patent Document 5]
   Japanese Unexamined Patent Publication No. 30093/2004 (*Tokukai* 2004-30093; published on January 29, 2004)
[Patent Document 6]
   Japanese Unexamined Patent Publication No. 175305/2002 (*Tokukai* 2002-175305; published on June 21, 2002)
[Patent Document 7]
   Japanese Unexamined Patent Publication No. 4739/2003 (*Tokukai* 2003-4739; published on January 8, 2003)

Conventionally, the array technique has been developed primarily for academic purposes centered on genome analysis, or for providing a research tool. As such, there has been no active development for more practical purposes. A problem of the array technique then is that it is not often suitable for practical purposes such as identification of individuals, or genetic analysis.

Specifically, in the array technique, the biosubstances or synthetic substances are immobilized on a support in an orderly fashion, but the order is not specific and the biosubstances or synthetic substances are randomly arranged in most cases. The random arrangement of biosubstances or synthetic substance, however, does not cause any problem as long as the array technique is used for the systematic and comprehensive analysis of genes, etc. That is, there was no special meaning in arranging the biosubstances or synthetic substances in a predetermined order based on some criteria.

However, the systematic and comprehensive analysis of genes, etc. has potential use in more practical applications such as variety improvement of plants, for example. In using the array technique for such purposes, it is desirable that the biosubstances and synthetic substances be analyzed with additional position information of chromosomes. In some cases, it may be required to use some kind of reference to set the order of arrangement.

A problem of the conventional bioinformatics technique is that it cannot be used to efficiently perform crossing for variety improvement, QTL analysis, and the like.

Specifically, in crossing, numerous numbers of individuals in the hybrid generations need to be screened for individuals in which target traits are expressed. Conventionally, it is been required to grow the hybrid generation for several years until the traits are confirmed. Further, depending on the type of trait, the traits cannot be easily recognized by simply growing the hybrid individuals. On the other hand, if the screening is performed with large gene expression data obtained from the nucleic acid array, whether target traits have been inherited or not can be efficiently confirmed with good reproducibility only by obtaining nucleic acids from the individuals of the hybrid generation.

However, since the conventional bioinformatics technique concerning gene expression is not intended for such a purpose, the gene expression data obtained from the DNA micro array has not been effectively used for crossing.

The QTL analysis involves statistical analysis. When only this aspect of QTL analysis is considered, the bioinformatics technique is easily applicable to the QTL analysis. However, no technique is known that uses the array technique and the bioinformatics technique in combination for the QTL analysis. Further, as to the conventional bioinformatics technique concerning gene expression, it has not been possible to effectively use the technique in the QTL analysis.

Further, while the conventional technique allows information concerning gene functions or regulating functions to be obtained by performing an expression profile analysis on cells of a particular type or particular stage, the technique cannot provide enough information concerning expression of genes associated with particular traits.

More specifically, since the expression profile analysis analyzes expression profiles of cells of a particular type or particular stage, a comprehensive gene expression analysis can be carried out and expression patterns specific to a particular cell type or particular cell stage can be obtained. However, while the technique is useful in finding target genes or a target gene group, it is not sufficient to analyze which genes or a group of genes are associated with predetermined specific traits or genes of interest.

That is, the comprehensive gene expression analysis is useful in finding clusters or networks in a vast amount expression information, and obtaining therefrom specific genes or a group of genes. However, the technique is not effective in analyzing which genes or a group of genes are associated with specific traits or genes of interest, because the technique in which a vast amount of expression information is narrowed down to desired information involves unnecessary information processing and may cause difficulties in accurately narrowing down the information.

### DISCLOSURE OF INVENTION

The present invention was made in view of the foregoing problems, and an object of the invention is to provide an array technique in which the order of arrangement of biosubstances or synthetic substances immobilized on a support is specified, and which is therefore applicable to, for example, screening in variety improvement of organisms.

Another object of the invention is to provide (1) a genotype analyzing and display system to be suitably used in effectively using gene expression data of a nucleic acid array in crossing for variety improvement, (2) a quantitative loci analyzing system to be suitably used in effectively using data of a nucleic acid array in QTL analysis, (3) a gene interaction analyzing system for effectively analyzing, using the result of analysis obtained from the nucleic acid array, which genes or a group of genes are associated with target traits or genes that have been specified beforehand, and (4) representative methods of using such analyzing systems.

The inventors of the present invention diligently worked to solve the foregoing problems, and accomplished the invention by finding that, for example, a DNA micro array can be used for screening in variety improvement of living organisms when DNA fragments immobilized on a glass substrate (support) are arranged in the order they are coded for on the chromosomes, or when information obtained from the array is analyzed with such order information.

In order to achieve the foregoing objects, the present invention provides an array in which different kinds of biosubstances obtained from an organism of interest, or synthetic substances interacting with such biosubstances are arranged and immobilized on a support in an orderly manner, the different kinds of biosubstances or the synthetic substances being arranged such that a chromosomal order of base sequence blocks corresponding to the biosubstances is ascertainable.

In one specific example of the array in which the biosubstances or synthetic substances are arranged in such a manner that their chromosomal order is recognizable, different kinds of biosubstances or synthetic substances are arranged in the chromosomal order of respective base sequence blocks of the biosubstances. Such an arrangement will be called a "direct arrangement" (see First Embodiment).

In the direct-arrangement array, it is not necessarily required that all of the biosubstances or synthetic substances are arranged in the chromosomal order of respective base sequence blocks of the biosubstances. As such, only some of the biosubstances or synthetic substances may be arranged in the chromosomal order of their respective base sequence blocks. The support may include labels that indicate the chromosomal order of the respective base sequence blocks of the biosubstances.

In another example of the chromosomal order recognizable array, the biosubstances or synthetic substances immobilized on the support are each appended with sequence position information corresponding to the chromosomal order of the respective sequence blocks of the biosubstances, and, in use, data is acquired and the sequence position information is read out, so as to rearrange sequences of the data in the chromosomal order. Such an arrangement will be called an "indirect arrangement" (see First Embodiment).

In a more specific example of the indirect-arrangement array, the support is realized by a collection of micro supports individually immobilizing the biosubstances or synthetic substances, and each micro support is appended with sequence position information corresponding to the chromosomal order of the respective base sequence blocks of the biosubstances. Based on the sequence position information, the order of acquired data is rearranged in the chromosomal order.

In the array, nucleic acids or polypeptides can be used as the biosubstances. The nucleic acid may be DNA, for example. The type of DNA is not particularly limited, but a genetic marker, genomic DNA, genomic DNA treated with restriction enzyme, cDNA, EST, and synthetic oligoDNA are preferably used, for example. It is preferable that a plurality of DNA molecules immobilized on the support be arranged based on a genetic map or physical map.

As a rule, in order to quantify gene expression level, cDNA or cRNA derived from mRNA is generally used as a target sample. In addition to cDNA and cRNA, the target sample used in an array of the present invention may be genomic DNA treated with restriction enzyme, when the biosubstance is nucleic acid. Here, it is preferable that the target DNA have been fractionated by size after treated with restriction enzyme.

When the biosubstance is polypeptide, proteins, fragments of proteins, or oligopeptides can be used as the biosubstances. The type of protein is not particularly limited. For example, enzymes, kinase, antibodies, receptors, and proteins with an SH3 region may be used. It is preferable that the proteins be arranged based on a genetic map or physical map (see Second Embodiment).

In an array according to the present invention, the support or micro support may be an inorganic substrate, an organic membrane, or a bead. More specifically, an array according to the present invention may be a micro array, a macro array, a bead array, or a protein chip.

A producing process of an array according to the present invention includes the step of orderly arranging and immobilizing on a support different kinds of biosubstances obtained from an organism of interest, or synthetic substances interacting with such biosubstances, the step including arranging and immobilizing the biosubstances or the synthetic substances according to the order in which genes corresponding to the biosubstances are coded for on a chromosome of the organism. In the process, nucleic acids or polypeptides may be used as the biosubstances.

Use of the present invention is not particularly limited. For example, the invention can be used for identification of a genotype, in which a chromosome fragment including a target trait is identified from hybrids obtained by crossing, with the use of an array using DNA as the biosubstance. The organism used for the identification of such a chromosome fragment is not particularly limited, and experimental animals and plants can be used, for example. Further, the organism used for this purpose may be a human. In this case, the genotype identification method can be used as a gene diagnosis method.

The present invention can also be used, for example, for screening in variety improvement, whereby a variety including a target trait is selected, with the use of an array using DNA as the biosubstance, from hybrids obtained by crossing of organisms whose characteristics are to be improved. Here, the type of organism used for variety improvement is not particularly limited. For example, domestic animals or crops can be used. Specific examples of crops include cereals such as rice, wheat, corn, and barley.

The inventors of the present invention diligently worked to achieve the foregoing objects, and accomplished the invention based on the following finding. Namely, the inventors found that, in analyzing gene expression data obtained from hybrid individuals with the nucleic acid array, use of at least (1) genetic information of parents of the hybrid individuals and (2) a genetic map of the species to which these individuals belong allows the gene expression data to be analyzed based on graphical representation of locations of crossovers on the chromosomes, and thereby enables the gene expression data obtained with the nucleic acid array to be effectively used in crossing for variety improvement.

Namely, a genotype analyzing and display system according to the present invention includes: a genotype origin detecting section for comparing (a) gene expression level information comprehensively obtained through a hybridization analysis of hybrid individuals with a nucleic acid array with (b) genetic information of parents of the hybrid individuals, and a genetic map of a species to which the hybrid individuals belong, so as to determine whether a genotype of a hybrid individual of interest derives from which parent; and a display information generating section for gathering a plurality of results obtained from the genotype origin detecting section and, based on the results, generating display information used to display a plurality of genotypes altogether on a chromosome basis, so as to determine whether individual genotypes derives from which parent (see Fourth Embodiment).

In the genotype analyzing and display system, it is highly preferable that the nucleic acid be a chromosomal location recognizable array in which a plurality of nucleic acid molecules immobilized thereon are arranged such that a chromosomal order of base sequence blocks corresponding to the nucleic acid molecules is ascertainable.

It is preferable that the genotype analyzing and display system includes a genetic map constructing section for constructing, based on genetic map constructing information, a genetic map of a species to which the hybrid individuals belong. It is preferable that the genetic map constructing information includes names of genes and/or genetic markers known in the species, and chromosomal loci of the genes and/or genetic markers.

In the genotype analyzing and display system, it is preferable that the genotype origin detecting section determines a genotype as being homozygous for one of the parents, heterozygous, or unrecognizable to yield a result. Further, it is preferable that the genotype origin detecting section use genotype information and/or gene expression profile information of parents as genetic information of parents.

In the genotype analyzing and display system, it is preferable that the display information generating section generate display information including at least one of recombination number and recombination frequency of individual chromosomes. Further, it is preferable that the display information generating section generate display information such that an origin of a genotype is identifiable based on different display colors or patterns.

It is preferable that the genotype analyzing and display system include at least one of an input section and an output section. The input section preferably receives at least one of comprehensive expression level information of genes of the hybrid individuals, and genetic information of parents. Further, the input section preferably receives genetic map constructing information.

The input section may be, for example, a scanner for enabling a hybridization result of the nucleic acid array to be read out as image information. Preferably, an image information processing section is also provided that analyzes an expression level of gene based on the image information and generating comprehensive expression level information of gene.

It is preferable that the input section be a manual input section for modifying at least one of: the comprehensive expression level information of gene of the hybrid individuals; the genetic information of parents; and the genetic map constructing information.

It is preferable that the output section include at least one of: a display for displaying the display information on a screen; and a printer for printing the display information. Preferably, the input section and output section are realized by an external communications section for sending and receiving information to and from an external device.

In the genotype analyzing and display system, the nucleic acid array is generally, but not limited to, a DNA array on which DNA is immobilized. Specific examples of DNA immobilized on the DNA array include a genetic marker, genomic DNA, genomic DNA treated with a restriction enzyme, cDNA, EST, and synthetic oligoDNA. Specific examples of the nucleic acid array include a micro array, a macro array, and a bead array.

Use of the present invention is not particularly limited. For example, the invention can be used for identifying a target trait-including chromosome fragment, using the genotype analyzing and display system, from hybrids obtained by crossing organisms. The organisms may be experimental animals and plants.

The invention can also be used for screening for a target trait-carrying variety from hybrids obtained by crossing organisms whose characteristics are to be improved, using the genotype analyzing and display system. The organisms crossed for variety improvement may be experimental animals and plants, domestic animals, or crops.

The inventors of the present invention diligently worked to achieve the foregoing objects, and accomplished the invention by finding that the gene expression data obtained with the nucleic acid array can be effectively used for the QTL analysis when the result of hybridization obtained from the spots of the nucleic acid array is used as genetic marker information.

Namely, a quantitative loci analyzing system according to the present invention include: a genetic marker specifying section for comparing (a) comprehensive presence information of genes of hybrid individuals, obtained by hybridizing a genomic sample of the hybrid individuals of a certain hybrid line with a nucleic acid array on which a genetic marker of a species of interest is immobilized (b) with a genetic map of a species to which the hybrid individuals belong, and genetic marker information known in the species, so as to specify a genetic marker that exists in the hybrid line; and a quantitative loci detecting section for detecting a quantitative locus of a phenotype of interest of the hybrid individual, by confirming whether a phenotypic value indicative of the phenotype is linked to the genetic marker (see Fifth Embodiment).

In the quantitative loci analyzing system, it is highly preferable that the nucleic acid array be a chromosomal location recognizable array in which a plurality of nucleic acid molecules immobilized thereon are arranged such that a chromosomal order of base sequence blocks corresponding to the nucleic acid molecules is ascertainable.

It is preferable that the quantitative loci analyzing system include a genetic map constructing section for constructing, based on genetic map constructing information, a genetic map of a species to which the hybrid individuals belong. The genetic map constructing information preferably includes names of genes and/or genetic markers known in the species, and chromosomal loci of the genes and/or genetic markers.

In the quantitative loci analyzing system, it is preferable that the genetic marker information used by the genetic marker specifying section include a genetic marker with polymorphism. More specifically, the genetic marker is preferably SNP or RFLP.

In the quantitative loci analyzing system, it is preferable that the quantitative loci detecting section detect a quantitative locus of phenotype by interval mapping.

It is preferable that the quantitative loci analyzing system include: a scanner for enabling a hybridization result of the nucleic acid array to be read out as image information; and an image information processing section for analyzing an expression level of gene based on the image information and generating comprehensive expression level information of gene.

It is preferable that the quantitative loci analyzing system include at least one of an input section and an output section. Here, the scanner can be used as an input section. The input section preferably receives at least one of the genetic marker information and the phenotypic value. Further, the input section preferably receives at least one of the genetic map and the genetic map constructing information.

Further, it is preferable that the input section be a manual input section for modifying at least one of: the comprehensive presence information of gene of the hybrid individuals; the genetic marker information, and the genetic map constructing information.

It is preferable that the output section be at least one of a display for displaying an analysis result on a screen; and a printer for printing an analysis result. Preferably, the input section and output section be realized by an external communications section for sending and receiving information to and from an external device.

In the quantitative loci analyzing system, the nucleic acid array is generally, but not limited to, a DNA array on which DNA is immobilized. Specific examples of the nucleic acid array include a micro array, a macro array, and a bead array.

Use of the present invention is not particularly limited. For example, the invention can be used as a quantitative trait analyzing method for analyzing a quantitative trait of an organism, using the quantitative loci analyzing system, or a gene searching method for searching for a gene associated with expression of a trait of interest, using the quantitative loci analyzing system, or a variety improvement method for organisms, which uses the quantitative loci analyzing system. The organisms used for variety improvement are preferably laboratory animals and plants, domestic animals, or crops.

The inventors of the present invention diligently worked to achieve the foregoing objects, and accomplished the invention by finding that an analysis of whether or not which gene or which group of genes is associated with a previously specified trait or gene of interest can be effectively performed when hereditary factors for regulating the expression level of individual genes are described based on the hybridization results of the genetic markers immobilized on the nucleic acid array.

Namely, a gene interaction analyzing system according to the present invention includes: a genetic marker specifying section for comparing (a) comprehensive presence information of genes of hybrid individuals, obtained by hybridizing a genomic sample of the hybrid individuals of a certain hybrid line with a nucleic acid array on which a genetic marker of a species of interest is immobilized (b) with a genetic map of a species to which the hybrid individuals belong, and genetic marker information known in the species, so as to specify a genetic marker that exists in the hybrid line; a spot marker information generating section for comparing the specified genetic marker with the genetic marker immobilized on the nucleic acid array, so as to generate spot marker information, being genetic marker information for use in analysis, from hybridization results obtained from individual spots on the nucleic acid array; and a hereditary factor specifying section for specifying, with regard to an arbitrarily selected phenotype and gene to be analyzed, a hereditary factor of the selected phenotype by determining whether the phenotypic value indicative of the phenotype, and an expressed gene included in expression profile information obtained from the hybrid individual are linked to a plurality of spot marker information (see Sixth Embodiment).

In the gene interaction analyzing system, it is highly preferable that the nucleic acid array be a chromosomal location recognizable array in which a plurality of nucleic acid molecules immobilized thereon are arranged such that a chromosomal order of base sequence blocks corresponding to the nucleic acid molecules is ascertainable.

It is preferable that the gene interaction analyzing system include a genetic map constructing section for constructing, based on genetic map constructing information, a genetic map of a species to which the hybrid individuals belong. Further, it is preferable that the genetic map constructing information be names of genes and/or genetic markers known in the species, and chromosomal loci of the genes and/or genetic markers.

In the gene interaction analyzing system, it is preferable that the genetic marker information used by the genetic marker specifying section be a genetic marker with polymorphism. More specifically, the genetic marker is preferably SNP or RFLP.

In the gene interaction analyzing system, the spot marker information generating section generates spot marker information only for a genetic marker spot found by hybridization. Here, it is preferable that the spot marker information generating section generate spot marker information by including position information of a genetic marker immobilized on the nucleic acid array.

It is preferable that the gene interaction analyzing system include an expression profile information generating section for analyzing an expression profile in regard to a comprehensive gene expression level obtained from the hybrid individual, so as to generate expression profile information of the hybrid individual. The expression profile information generating section generates expression profile information of the hybrid individual by comprehensively measuring gene expression, using at least one of a micro array, a macro array, a bead array, and a differential display. Here, it is preferable that the expression profile information generating section generate expression profile information using a nucleic acid array used to obtain comprehensive presence information of gene of the hybrid individual, or a nucleic acid array on which the sample has been spotted.

The DNA array on which DNA is immobilized can be suitably used as the nucleic acid array for obtaining the gene-presence-information, or the nucleic acid array for obtaining expression profiles. Specifically, the nucleic acid may be a micro array, a macro array, or a bead array.

In the gene interaction analyzing system, the hereditary factor specifying section specifies a hereditary factor of a phenotype based on a quantitative trait locus (QTL) that exists among genetic markers obtained by interval mapping. Here, the hereditary factor specifying section may uses information of expression level of a gene associated with the genetic marker, so as to specify a hereditary factor of the phenotype.

The gene interaction analyzing system includes at least one of an input section and an output section. The input section receives at least one of: comprehensive presence information of gene of the hybrid individual; the genetic marker information; the phenotypic value; and the expression profile information. Preferably, the input section receives at least one of the genetic map and the genetic map constructing information.

The input section is not limited to a particular structure. For example, the input section may be provided as a scanner for enabling a hybridization result of the nucleic acid array to be read out as image information. Here, it is preferable that an image information processing section be provided that analyzes an expression level of gene based on the image information and generating comprehensive expression level information of gene. The scanner may be used as an input section for entering the expression profile information.

Further, it is preferable that the input section be provided as a manual input section for modifying at least one of: the comprehensive presence information of gene of the hybrid individuals; the genetic marker information, and the genetic map constructing information.

It is preferable that the output section be at least one of a display for displaying an analysis result on a screen; and a printer for printing an analysis result. Further, it is preferable that the input section and the output section be realized by an external communications section for sending and receiving information to and from an external device.

Use of the present invention is not particularly limited. For example, the present invention may be used as a gene interaction analyzing method for analyzing interaction between genes, using the gene interaction analyzing system, or a gene searching method for searching for a gene associated with a trait of interest, using the gene interaction analyzing system, or a variety improvement method for organisms, which uses the gene interaction analyzing system. The organisms used for variety improvement may be laboratory animals and plants, domestic animals, or crops.

For a fuller understanding of the nature and advantages of the invention, reference should be made to the ensuing detailed description taken in conjunction with the accompanying drawings.

### BRIEF DESCRIPTION OF DRAWINGS

Fig. 1 is a schematic diagram showing a specific exemplary structure of an array according to the present invention, when the substance immobilized on a support (substrate) is DNA.
Figs. 2(a) and 2(b) are plan views schematically illustrating expression of genes with particular characteristics in the array of Fig. 1.
Fig. 3 is a schematic diagram showing expression of genes with particular characteristics, concerning a resulting segregating population of the cross between varieties respectively expressing genes as shown in Figs. 2(a) and 2(b), and a specific variety selected from the segregating population.
Fig. 4 is a schematic diagram showing a specific exemplary structure of an array according to the present invention, when the substance immobilized on a support (substrate) is protein.
Fig. 5 is a schematic diagram showing a specific exemplary structure of an array according to the present invention, when the substance immobilized on a support (substance) is a compound (synthetic substance) which specifically interacts with protein.
Fig. 6 is a schematic diagram showing a specific exemplary structure of a bead array as one example of an array according to the present invention.
Fig. 7 is a block diagram illustrating an example of a genotype analyzing and display system according to the present invention.
Fig. 8 is a view illustrating an example of display information displayed in the genotype analyzing and display system according to the present invention.
Fig. 9 is a flowchart representing an example of an analysis method employed by the genotype analyzing and display system according to the present invention.
Fig. 10 is a block diagram illustrating an example of a quantitative loci analyzing system according to the present invention.
Fig. 11 is a flowchart representing an example of an analysis method employed in the quantitative loci analyzing system according to the present invention.
Fig. 12 is a block diagram illustrating an example of a gene interaction analyzing system according to the present invention.
Fig. 13 is a flowchart representing an example of an analysis method employed by the gene interaction analyzing system according to the present invention.

### BEST MODE FOR CARRYING OUT THE INVENTION

### [First Embodiment]

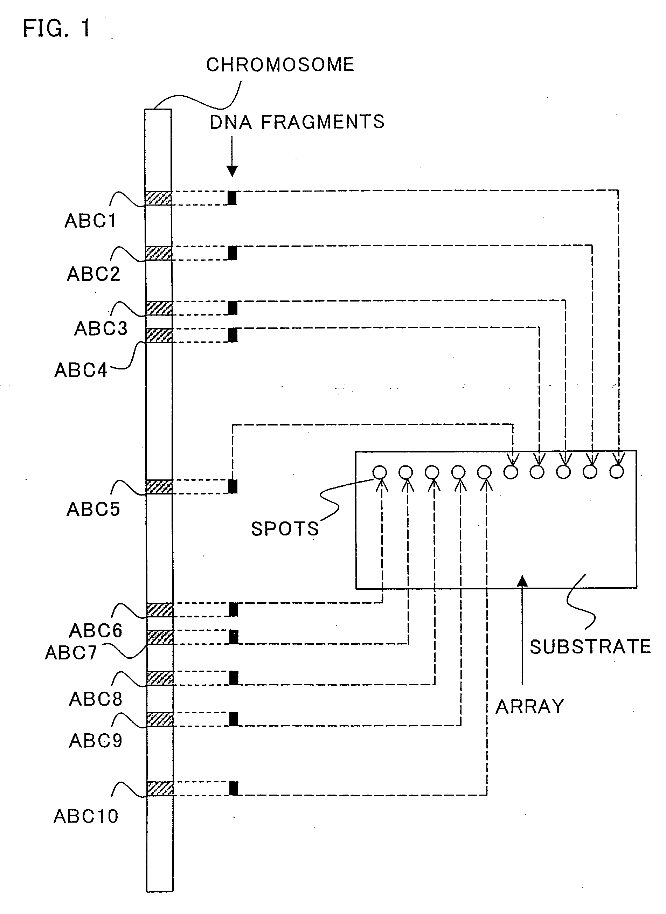

The following will describe one embodiment of the present invention with reference to Fig. 1 through Fig. 3. It should be appreciated that the present invention is not just limited to the particular embodiment described below.

According to the present invention, there is provided an array in which substances are immobilized on a support by being arranged in a chromosomal order. The invention is applicable to a wide range of array techniques. As used herein, the "array techniques" refer to techniques concerning arrays in which different kinds of substances are orderly arranged and immobilized on a support.

An array according to the present invention can be classified according to the type of substance immobilized, the type of support, use, or the like. The invention, to a large extent, is characterized by the order of substances immobilized on a support, and therefore the following specifically describes representative examples of the invention based on different types of substances immobilized on a support. First, in the present embodiment, the invention will be described through the case where the substance immobilized on a support is nucleic acid.

### <Basic structure of array>

The basic structure of an array used in the present invention is not particularly limited. As noted above, the invention provides an array in which a substance is immobilized on a support. Here, the support (substrate) is not particularly limited and may have any shape and may be made of any material as long as it can immobilize the substance.

Examples of support materials include, generally, inorganic materials such as glass or silicon wafer; natural polymers such as paper; synthetic polymers such as nitrocellulose or nylon; and gels using synthetic polymer or natural polymer. The shape of the support is not particularly limited either as long as it has a sufficient area on which the substance can be immobilized. Generally, those with a two dimensional plane, for example, such as a substrate with little or no flexibility, a flexible membrane, or a flexible substrate with intermediate flexibility can be preferably used. The thickness of the substrate or membrane is not particularly limited either, and it can be suitably set according to the material or use of the substrate or membrane.

The invention can also use bead arrays, as will be described later. As such, the support may be a collection of micro-supports on which biosubstances or synthetic substances are individually immobilized. As such micro-supports, various beads may be used, for example.

Here, a collection (group) of micro-supports makes up a single support. Such a group of micro-supports is prepared and used as a dispersion liquid (or a solution) charged into a small container, in which micro-supports immobilizing biosubstances (nucleic acid, protein, etc.) are dispersed. In this way, data can be freely acquired from the micro-supports. Each micro-support is appended with an ID code, and data is acquired from the micro-support with the ID code. Thus, the order of substances immobilized on the micro-supports corresponds to the arranged order of data acquired from the micro-supports based on the ID codes.

As used herein, the "substances immobilized on a support" refer to different kinds of biosubstances obtained from a living organism of interest, or synthetic substances which interact with such biosubstances. In other words, in an array according to the present invention, it is required that the substances immobilized on a support be at least substances associated with biosubstances derived from living organisms. Substances which are not associated with biosubstances cannot be used because, in this case, the coding order of chromosomes cannot be used as a basis of arranging these substances.

Nucleic acids and polypeptides are specific examples of such biosubstances. As nucleic acids, DNA and RNA can be used. Use of polypeptides as the biosubstances will described in detail in the Second Embodiment. As to use of synthetic substances that interact with biosubstances, detailed description will be given in the Third Embodiment. Note that, the biosubstances may include sugar chains, etc.

In an array according to the present invention, different kinds of biosubstances or synthetic substances are arranged in such a manner that the chromosomal order of respective base sequence blocks of these biosubstances is recognizable. Thus, for convenience of explanation, an array according to the present invention will be referred to as a chromosomal location recognizable array. In one specific implementation of such a chromosomal location recognizable array, different kinds of biosubstances are arranged in the chromosomal order. For convenience of explanation, such an arrangement will be called a "direct arrangement," because the order of the substances arranged on the array directly corresponds to the order in which these substances are sequenced on the chromosome.

In another implementation of a chromosomal location recognizable array, the order of the substances arranged on the array indirectly corresponds to the chromosomal order. This will be called an "indirect arrangement."

### <Direct-Arrangement Array>

The present embodiment is described below in more detail based on an example (direct-arrangement array) in which DNA, as an example of nucleic acid, is arranged on a support in a chromosomal order.

For example, it is assumed here that an array is fabricated for an organism Z based on an organism Z chromosome in which 10 genes ABC1 through ABC10 are present that are lined up in this order on the chromosome, as schematically illustrated in Fig. 1. It is also assumed that the genes ABC1 through ABC 10 respectively have corresponding DNA fragments (assuming that such DNA fragments are obtained). In this case, an array is fabricated by spotting these DNA fragments in an orderly manner on a substrate. Note that, in the following, the biosubstances immobilized on a substrate will be referred to as "spots" where appropriate.

In spotting the DNA fragments on the substrate, a device called a spotter or arrayer is generally used. The operation of the spotter is controlled in such a manner that the DNA fragments are spotted in the order their corresponding genes are found on the chromosome. In this way, the DNA fragments are immobilized on the support by being arranged in the order "respective base sequence blocks of the biosubstances are sequenced on the chromosome."

As used herein, the "base sequence block" refers to a region of a certain length in the base sequence of a chromosome. A typical example is a region corresponding to a gene that encodes a protein. It should be noted, however, that the "base sequence block" is not just limited to gene but may be a large DNA fragment like a BAC (Bacterial Artificial Chromosome) clone, or a region corresponding to only an exon. Further, the "base sequence block" may be a region, like EST, that does not necessarily include a coding region of a protein.

Referring to the foregoing example, the chromosomal order may be simply the order of the genes ABC1, ABC2, ABC3, ... up to ABC10, or the order of three different fragments of ABC1 gene, three different fragments of ABC2 gene, and three different fragments of ABC3 gene, and so on. Here, the number of fragments may be three for ABC1 gene, two for ABC2 gene, and five for ABC3 gene. Namely, the order of substances immobilized on the support is not particularly limited as long as, when taken as a whole, it corresponds to the order in which these substances are sequenced on the chromosome.

In the example illustrated in Fig. 1, a plurality of DNA fragments occurs on a single chromosome. However, the present invention is not just limited to this example, and the DNA fragments may occur in more than one chromosome. In this case, as with the foregoing, the DNA fragments are arranged on the array in the order they are sequenced on the chromosomes.

Further, in the example illustrated in Fig. 1, a plurality of DNA fragments is arranged as they are sequenced on the chromosome. However, the present invention is not just limited to this example. For example, in order to meet different purposes, only some of the DNA fragments may be arranged in the chromosomal order. That is, an array according to the present invention may immobilize substances other than nucleic acids, and at least some of the different kinds of biosubstances or synthetic substances may be arranged in the order the respective base sequence blocks of the biosubstances are sequenced on the chromosome.

Further, in the direct arrangement, the chromosomal order can be recognized by techniques other than arranging the substances in the chromosomal order. For example, labels indicative of the chromosomal order of respective base sequence blocks of the biosubstances may be appended on the support.

As an example, labels may be provided that can distinguish between first and second rows of DNA fragments obtained from an organism of interest, wherein the first row includes 10 kinds of DNA fragments (spots) obtained from chromosome 1 and arranged in the chromosomal order, and the second row includes 10 kinds of DNA fragments (spots) obtained from chromosome 2 and arranged in the chromosomal order. Further, as in the indirect arrangement described below, information indicative of the type of DNA fragment immobilized on each spot may be appended as a label in the vicinity of each spot.

### <Indirect-Arrangement Array>

The following describes the indirect-arrangement array. In the indirect-arrangement array, sequence position information corresponding to the chromosomal order of the base sequence blocks of the biosubstances is added to each of the biosubstances or synthetic substances immobilized on the support. This enables acquired data to be rearranged in the chromosomal order based on the sequence position information, irrespective of the order of the immobilized substances.

A specific example of the indirect-arrangement array is a bead array, in which the support is a collection of micro-supports individually immobilizing biosubstances or synthetic substances (bead array will be described later). In this arrangement, each micro-support is appended with sequence position information corresponding to the order in which respective base sequence blocks of the biosubstances are sequenced on the chromosome.

In use, data is acquired and the sequence position information is read out. Based on the sequence position information, the sequence of the acquired data is rearranged in the chromosomal order. By thus recognizing the chromosomal order, the substances immobilized on the micro-supports can be arranged in the chromosomal order.

Note that, a specific form of the sequence position information is not particularly limited as long as it corresponds to the chromosomal order of the respective base sequence blocks of the DNA immobilized on the micro-supports.

### <Immobilized DNA>

In the present embodiment, DNA is used as the biosubstance. The type of DNA (DNA fragment) is not particularly limited, but a genetic marker, genomic DNA, genomic DNA treated with restriction enzyme, cDNA, EST, and synthetic oligoDNA are preferably used, for example. It is preferable that the DNA be arranged based on a genetic map or physical map. For example, for a group of different kinds of genetic markers, it is preferable that these genetic markers make up a genetic map. Based on the genetic map, the DNA fragments can be arranged on a substrate.

The genetic marker or a group of genetic markers are not particularly limited as long as they can serve as genetic labels on the chromosome. Non-limiting examples include an EST marker using EST, a SNP marker including SNP (Single Nucleotide Polymorphism), a RFLP (Restriction Fragment Length Polymorphism) marker, and a micro satellite marker (SSR (simple sequence repeat) marker). Thus, the genetic marker or a group of genetic markers include genomic DNA treated with restriction enzyme, EST, synthetic oligoDNA, and the like, if they can be used as markers.

The number of biosubstances immobilized on the support is not particularly limited, and it is generally on the order of several thousand (10³). The number of immobilized (or arranged) biosubstances varies greatly depending on the type of device, such as a spotter, used for the fabrication of the array, or the area of the support (substrate), for example.

It should be noted that, in the DNA array, information concerning gene expression can only be obtained for genes corresponding to the immobilized DNA fragments. It is therefore preferable to increase the number of immobilized biosubstances (DNA fragments) as much as possible, in order to perform gene expression analysis more systematically and comprehensively.

### <Types of arrays used in the embodiment>

The type of array used in the present invention is not particularly limited and various conventional arrays can be used. Specifically, a micro array, a macro array, a bead array, or a protein chip can be used, for example. The present embodiment uses nucleic acid as the biosubstance, and therefore more specific examples include a DNA micro array and DNA macro array, for example.

The DNA micro array is also known as a DNA chip, and the immobilized DNA is often referred to as a probe. The micro array is smaller in size than macro array and provides more density. This enables the number of genes (DNA fragments) immobilized as probes to be increased, allowing for more comprehensive gene expression analysis.

The DNA micro array can be classified based on types of immobilized DNA. However, structural differences can be revealed more clearly if the DNA micro array is classified based on fabrication methods. Specifically, based on fabrication methods, the micro array can be broadly classified into the Stanford type and the Affymetrix type.

A DNA micro array of the Stanford type is fabricated by spotting a DNA solution onto a substrate (support) with a spotter, wherein a slide glass for a microscope is used as the substrate. One advantage of a DNA micro array of a Stanford type is that it can always be fabricated with the use of a spotter. However, this comes with a drawback in that it requires expensive hardware (spotter, etc.), or complex procedures for the preparation of biosubstances as necessitated by a large number of probes required for spotting.

On the other hand, a DNA micro array of the Affymetrix type, as described in the BACKGROUND ART section, does not employ the method of immobilizing DNA fragments on a substrate with a spotter, etc., but is fabricated by chemically synthesizing oligoDNA of about 25 mer on a substrate using a micro fabrication technique commonly used in the fabrication of semiconductors, namely, a photolithography technique.

Specifically, for each gene, 11 to 20 oligos (25 mers) (for example, 11 oligos in the case of a barley DNA array) are set based on base sequence data, and a pair of oligo DNA: one with a perfect match to each 25 mer, and one with a forced single-base mismatch at the 13th base is used as a probe. The array can be fabricated without using a spotter or other devices when it is designed with data of a known database. Further, since the probe (DNA fragment) has a constant length and the sequence is known, the CG content, which influences the strength of hybridization, can remain constant. It should be noted, however, that since the probe is synthesized based on information of a database, clones to be analyzed need to be separately isolated.

As described above, the present invention is characterized by the order information of the sequence of the nucleic acids (biosubstances) immobilized on a substrate (support), and the invention can use various types of DNA, including synthetic oligoDNA, as the nucleic acids. This makes the techniques of the present invention suitable for both Stanford DNA micro array and Affymetrix DNA micro array.

The following describes an exemplary method of using the DNA micro array. First, the DNA micro array is hybridized with fluorescent-labeled target DNA (hereinafter, "targets"). Here, the target molecules containing complementary sequences to the probes on the DNA micro array bind to (hybridize with) their complementary probe molecules, leaving other target molecules unbound. Then, these target molecules not bound to the probes are washed and removed, leaving only the hybridized target molecules on the micro array. Since the target molecules are fluorescence-labeled, the fluorescence of the targets is measured as signal intensity and hybridized probes are identified.

The fluorescent-labeled targets are generally prepared first by extracting mRNA from cells of two different states (first state and second state) to be compared, and then performing a reverse transcription reaction in the presence of fluorescent nucleotides. Here, two kinds of fluorescent dyes with different detection wavelengths are used for the first state and second state, respectively. The expression level of genes is greater for the cDNA contained in the targets, and the fluorescent signal intensity is in accord with the expression level of genes in each state. Thus, from the measured signal intensity, the expression level of a specific gene can be detected.

The DNA macro array basically has the same structure as the DNA micro array, but differs from the DNA micro array in that it uses a common membrane filter like a nylon membrane as a substrate. An advantage of the macro array is that it allows for an expression profile analysis, genome wide, according to methods based on conventional blotting methods. Another advantage is that, unlike the micro array, the DNA does not detach in washing, owning to the fact that the spotted DNA is immobilized on a membrane filter after denatured by an alkali treatment. Therefore, the macro array and micro array should be suitably selected according to use.

The following describes an exemplary method of using the macro array. The macro array is used basically in the same way as the micro array. Specifically, the macro array is hybridized with isotope (³³P, etc.)-labeled targets. Then, target molecules that did not bind to the array are washed and removed, leaving only hybridized target molecules on the macro array. Here, since the target molecules are isotope-labeled, the spots are exposed on an imaging plate and the expression level of the targets is determined by measuring signal intensity from the imaging plate--a procedure not performed in the micro array.

The techniques of the present invention can also be applied to the mass array. In the mass array, genomic DNA fragments are arranged and immobilized in an orderly manner on a silicon substrate, and therefore the structure is basically the same as that of the micro array. The mass array was developed for SNP analysis, and as such it is used differently from the DNA micro array.

Specifically, oligonucleotides corresponding to regions in the vicinity of target SNP are synthesized and hybridized with the mass array. Then, by using the oligonucleotides as primers, a DNA fragment having a SNP single base difference is synthesized through elongation catalyzed by DNA polymerase. The DNA fragment is eluted and then ionized with MALDI. The SNP type can be determined by detecting a single base mass difference using TOS-MS. Note that, as to the MALDI-TOS-MS, details will be described later in the Third Embodiment.

The DNA micro array and macro array are both direct-arrangement arrays, whereas the bead array is classified as an indirect-arrangement array. The bead array is used in such a manner that, in a small container, a probe such as a nucleic acid or antibody is immobilized on a surface of each bead to which an ID code has been added, and that the probe immobilized on the probe surface is specified by reading the ID code of the bead. With use of a two-wavelength laser beam, 100 kinds of beads can be quantified. That is, in an array according to the present invention, the support may be a collection of micro arrays (beads, for example) on which biosubstances or synthetic substances are individually immobilized.

In applying the invention to the bead array, each bead is appended with an ID code containing sequence position information, as described above. In this way, measurement can be performed in the same manner as in the other techniques. Further, since the bead array allows for detection in a liquid phase, it is effective in efficiently quantifying proteins in particular. This will be described in detail in the Third Embodiment.

### <Target DNA>

The target DNA is not particularly limited. In quantifying the expression level of genes, cDNA or cRNA derived from mRNA is generally used as a target sample. In the present invention, genomic DNA treated with restriction enzyme can also be used, for example.

A gene expression analysis with a common DNA array (represented by DNA micro array) is based on the principle of Northern blotting. This is effective in detecting genes having different expression patterns between two samples that differ from each other by the presence or absence of a particular disease, for example. However, if the purpose of the analysis is to detect genetic differences between the two samples, finding different gene expression is often not effective in meeting such a purpose because different gene expression does not necessarily mean that the samples are genetically different.

For a comparative expression analysis of a large number of samples (lines) using known DNA micro array techniques, a strict coordination (synchronization) of growth stage is required between tested samples, or only specific tissues need to be collected. Further, since the mRNA (cDNA) used as target DNA is a collection of expressed genes, comparison can only be made for the information of genes whose expression is specifically activated or suppressed in a tested growth stage.

Further, there have been many reports that suggest difficulties of a DNA micro array analysis in detecting a specific mutated gene in the genome even if it is present, owning to the fact that the expression level may not reflect the amount of transcripts, that the genes may be expressed only in limited tissues or stages, or that the amount of transcripts may be too small to be detected by the Northern blotting method.

Meanwhile, diversity of genes is not necessarily governed by mutations in the coding regions of genes. For example, there have been many reports that address the presence or absence of insertion and/or deletion in the introns, or structural differences (for example, differences in promoter activities) in the expression regulating region like a promoter sequence.

One applicable area of the present invention is variety improvement. In this application, cereals can be suitably used for variety improvement, for example. Among cereals, the genome size of barley for example is greater than that of rice by more than 10 fold. It is then highly likely that the non-coding regions, which account for the majority of the barley genome, contribute to the intraspecies diversity in barley.

In a DNA array according to the present invention, the DNA fragments (biosubstances) immobilized on a support are arranged in the chromosomal order. Thus, with an array of the present invention, the location of chromosomal recombination can be grasped by a single round of testing. Thus, in an analysis using an array of the present invention, target DNA is prepared so as to allow for use of the Southern blotting method. In this way, structural mutations in the non-coding regions of genes can also be efficiently detected, in addition to solving the conventional problems associated with the Northern blotting method.

The method by which target DNA is prepared for Southern blotting is not particularly limited, and genomic DNA is fragmented by known methods. Specifically, genomic DNA subjected to restriction enzyme is used as target DNA. In other words, RFLP analysis is performed with an array of the present invention.

Digestion of genomic DNA with restriction enzymes produce probe DNA fragments of many different sizes as compared with using mRNA (cDNA). This can be a drawback where accurate detection of polymorphism, such as a length difference, for example, between 500 bp and 5 kbp is required on the array (detection sensitivity of imaging means for detecting image information of array is brought into question).

In order to avoid such a problem, DNA fragments obtained by the treatment of genomic DNA with restriction enzymes are fractionated by size to be used as target DNA. In this way, a length difference can be effectively detected as a polymorphism, enabling an array of the present invention to be effectively used in the analysis employing the Southern blotting method.

The method of size fractionation is not particularly limited, and any technique can be used as long as the method allows the genomic DNA treated with restriction enzymes to be fractionated to required sizes. For example, a commercially available nucleic acid purification column kit using a centrifugal tube can be used. Further, size fractionation can be performed by setting PCR conditions such that DNA fragments of certain sizes are specifically amplified. The labeling method of genomic DNA is not particularly limited, and labeling can be made by a known method using PCR, for example.

### <Fabrication method of array>

A fabrication method of array according to the present invention at least includes the step of arranging and immobilizing on a support different kinds of biosubstances obtained from a living organism of interest, or synthetic substances interacting with such biosubstances. In the step, the biosubstances or synthetic substances immobilized on the support are arranged in the order genes of the organism are coded on the chromosome.

When the biosubstances are nucleic acids as in the present embodiment, the step follows the following procedure, for example. After preparing genomic DNA, the genomic DNA is fragmented by restriction enzymes, and a solution of DNA fragments is spotted on the support using a spotter. Here, the DNA fragments are spotted with a spotter in such a manner that chromosome information of the corresponding genes can be identified, as described above.

The spotter is not particularly limited and known instruments can be suitably used. Specifically, for example, an instrument that sputters a DNA solution onto a substrate through a capillary pen, or an ink jet device that plots a DNA solution on a substrate is available.

In the case where the support is a collection of micro supports (beads) like a bead array (micro support group), DNA or other substances are individually immobilized on the beads, and sequence position information indicative of chromosomal locations of the immobilized DNA is added, together with an identification code, to each bead. The group of beads so obtained is dispersed in a known liquid to prepare a bead solution, which is then charged into a small container and used as a bead array.

### <Use of an array according to the invention>

Use of an array according to the present invention is not particularly limited. For example, in the case of an array using DNA as biosubstances, the array can be suitably used to identify chromosome fragments including a target trait (identification of genotype), from hybrids obtained by crossing living organisms. Further, the array can be suitably used to screen for a variety with a target trait, from hybrids obtained by crossing organisms for variety improvement.

In conventional arrays, the DNA fragments immobilized on the support are randomly arranged. This enables the expression level or other profiles of the immobilized DNA fragments to be individually analyzed. In hybrids, individual genes are inherited in units of blocks, from a point of crossing over to the next point of crossing over, on the chromosomes. Therefore, for the genotype identification or selection in variety improvement, etc., it is necessary to determine the location and extent of recombination and the presence or absence of unnecessary recombination, in addition to finding individual traits. Thus, conventional arrays with randomly arranged DNA fragments cannot be used efficiently for the screening in variety improvement, etc.

On the other hand, in an array of the present invention, the DNA fragments (biosubstances) immobilized on the support are arranged in the chromosomal order. Thus, with an array of the present invention, the location of recombination on the chromosomes can be found, if any, with a single round of testing. This allows for accurate selection of individuals with desirable traits from a segregating population of hybrid individuals. Further, with an array according to the present invention, chromosomal recombinations in the hybrid generation can easily be estimated. This allows a group of genes to be introduced in units of blocks, or genes in the blocks to be modified.

Further, with an array according to the present invention, the recombination patterns, i.e., the location and type of recombination on the chromosomes can be accurately grasped. Thus, by identifying conserved regions of chromosomes where recombination frequency is small in the population of hybrids or natural population, recombination can be efficiently promoted only in these regions of the chromosomes.

In an analysis using conventional arrays, the cause of signal failure at a particular spot, whether it is actually caused by unexpressed genes, or due to experimental error, cannot be accurately determined unless it is rechecked. In contrast, in an array according to the present invention, such an experimental error can easily be found because the chromosomal order of DNA fragments (biosubstances) immobilized on the support can be recognized from their arrangement.

For example, consider the situation where signals are obtained from spots in front of and after the spot where signal failure has occurred. In an array according to the present invention, the spots are arranged in such a manner that their chromosomal order is recognizable. As a rule, in order for a gene flanked by another gene on the same chromosome to be recombined, two recombinations must occur in close proximity. Given a significantly low probability of such a phenomenon, the signal failure can be attributed to experimental error. Thus, with an array according to the present invention, whether signal failure that has occurred at a particular spot is due to experimental error or not can easily be found, with the result that analysis accuracy is improved.

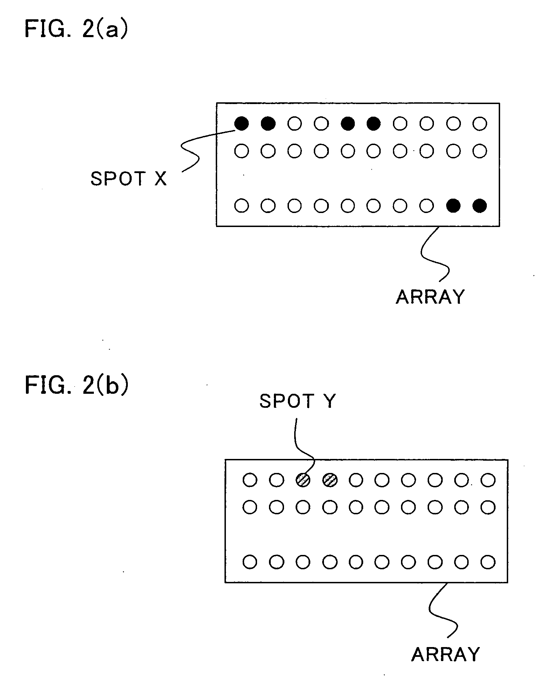

The following schematically describes an example of a screening method using an array according to the present invention. It is assumed here that a DNA micro array according to the present invention is fabricated using DNA fragments obtained from barley. In a DNA micro array according to the present invention, solid spots X in Fig. 2(a) indicate that genes that confer brewing characteristics are expressed, and hatched spots Y in Fig. 2(b) indicates expression of genes that confer disease-resistance.

In a DNA micro array according to the present invention, the spots are arranged in a chromosomal order, and therefore the positions of spots X and Y are fixed. For example, in Fig. 2(a), the spots X are fixed at the first, second, fifth, and sixth positions of the first row, and at the ninth and tenth positions of the bottom row. The spots Y are fixed at the third and fourth positions of the first row, as shown in Fig. 2(b).

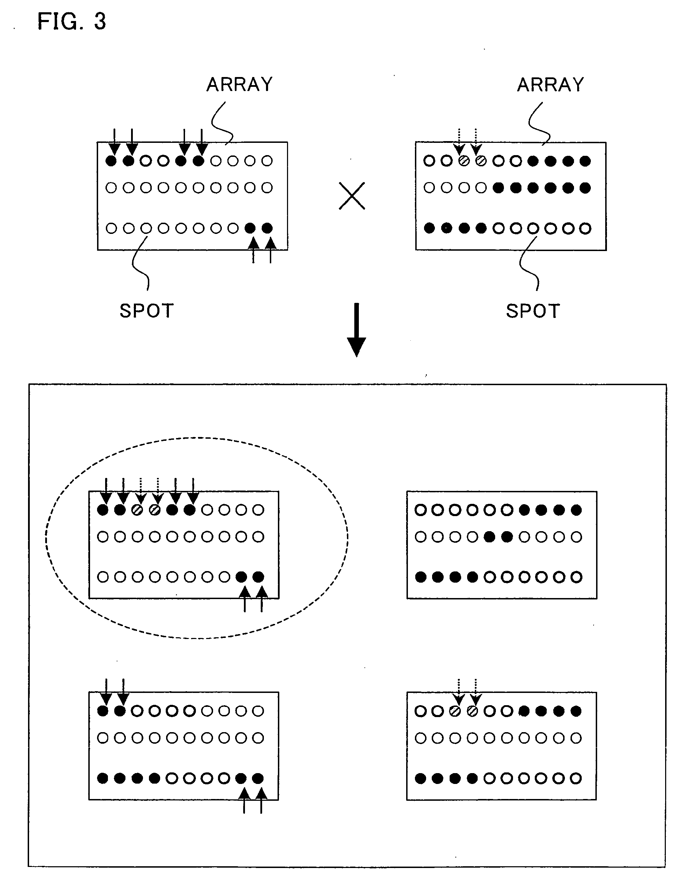

It is assumed here that segregating populations as represented by four micro arrays in the bottom of Fig. 3 were obtained from the cross between a variety expressing the brewing genes as indicated by spots X (corresponding to the upper left DNA micro array in Fig. 3) and a variety expressing the disease-resistant genes as indicated by spots Y (corresponding to the upper right DNA micro array in Fig. 3), for example. From the result of analysis using these DNA micro arrays, varieties expressing both the brewing genes and disease-resistant genes can be screened for from the segregating populations (variety corresponding to the upper left DNA micro array circled by a dotted line in the lower portion of Fig. 3).

Further, whether the chromosome fragments have derived from which parent can easily be determined also for other regions of the genome. Thus, a backcross, for example, between a hybrid and the variety shown in Fig. 2(a) easily allows for selection and growth of varieties having all of the expressed spots as illustrated in Fig. 2(a), i.e., the first, second, fifth, and sixth spots of the first row, and the ninth and tenth spots of the bottom row, as well as the third and fourth spots of the first row as shown in Fig. 2(b).

The type of organism to which an array of the present invention is applicable is not particularly limited, and any of plants, animals, and microorganisms may be used. Particularly, an array of the present invention can be used in the foregoing screening method in organisms that include chromosomes and obey the laws of Mendelian genetics. Examples of such an organism are, but not limited to, those commercially available and for which need for variety improvement is high.

In the case of plants, various crops (plant and farm products produced in agriculture, forestry, and fishery industries) can be used. Specific examples include: cereals such as rice, wheat, barley, rye, triticale, and corn; marine plants such as seaweed; various vegetables and flowers; and trees such as cedar or cypress. In the case of animals, various domestic animals can be used. Specific examples include: domestic mammals such as bovines, sheep, and pigs; domestic birds such as chickens and quails; fish such as yellowtail snapper, sea bream, carp, and sweetfish; insects such as honey bees, and silkworm; and shellfish such as oyster, ormer, and scallop. As microorganisms, bacteria such as *Escherichia coli,* yeasts, fungi, actinomycetes, and basidiomycetes can be used.

Among these examples, the cereals include crops such as rice, wheat, corn, and barley, which are cultivated worldwide and are strategically important. Thus, by using the present invention for the variety improvement of these plants, varieties with desirable traits can be efficiently produced.

An array according to the present invention can also be used for experimental animals and plants. Specific examples of experimental animals include mice, rats, D. *melanogaster,* and *C. elegans*. A specific example is *Arabidopsis thaliana*.

Further, for the purpose of identifying genotypes with an array of the present invention, the invention can be applied to humans. In other words, an array according to the present invention can be preferably used for a gene diagnosis method, since the array allows for efficient identification of genotypes.

### [Second Embodiment]

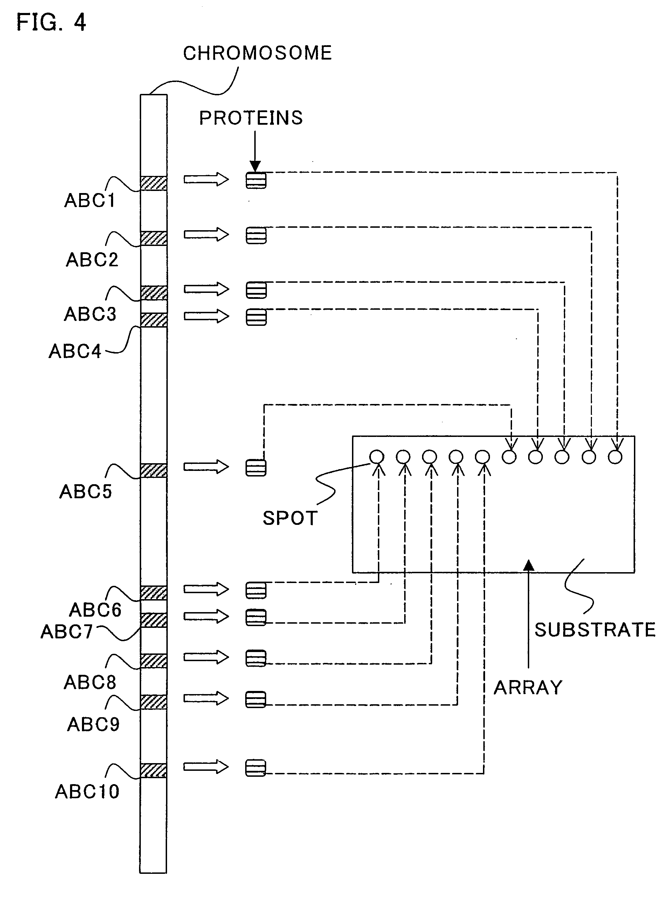

Referring to Fig. 4, the following will describe another embodiment of an array according to the present invention. It should be appreciated that the invention is not limited by the following description.

In the First Embodiment, the invention was described through the case where nucleic acids were used as the biosubstances. The present invention is not just limited to this example, and the biosubstances may be polypeptides.

### <Polypeptides as biosubstances>

Polypeptides used in the present embodiment are not particularly limited as long as they are peptides of amino acids. Specific examples are proteins, fragments of proteins, and oligopeptides. As used herein, "fragments of protein" refers to polypeptides of partial amino acid sequences of a complete protein. The "oligopeptide" refers to an oligopeptide with a molecular weight of no more than 5000. The "protein" includes a protein complex forming multimers, as well as monomer proteins.

In an array according to the present embodiment, as in the First Embodiment, the polypeptides immobilized on a support are arranged in the order respective base sequence blocks of the polypeptides are sequenced on a chromosome.

For example, it is assumed here, as in the example of Fig. 1, that an array is fabricated for an organism Z based on an organism Z chromosome in which 10 genes ABC1 through ABC 10 are present that are lined up in this order on the chromosome, as schematically illustrated in Fig. 4. It is also assumed that 10 kinds of proteins are respectively transcribed and translated from the genes ABC1 through ABC 10 (as indicated by arrows). In this case, an array is fabricated by spotting these proteins on a substrate in the chromosomal order.

The "base sequence blocks" may be regions corresponding to genes encoding the proteins, or regions corresponding to only polypeptides as fragments of protein.

The type of protein used as the biosubstance is not particularly limited. For example, enzymes, kinase, antibodies, and proteins with an SH3 region may be used. It is preferable that the proteins, as with the DNA in the First Embodiment, be arranged based on a genetic map or physical map.

### <Types of arrays used in the embodiment>

The type of array used in the present embodiment is not particularly limited as long as the polypeptides immobilized on the support are arranged in the order respective base sequence blocks of the polypeptides are sequenced on the chromosome. Specifically, for example, a peptide array, a kinase array, an enzyme array, an SH3 domain array, and a receptor array may be used, depending on the type of polypeptide immobilized on the support.

In the peptide array, oligopeptides are immobilized on a support. The oligopeptides may be synthetic, or may be obtained by degrading or cutting proteins or other polypeptides by a known method.

In the kinase array, different kinds of purified kinase proteins are arranged and immobilized on a support. By finding phosphorylation patterns exhibited by the kinase, the behaviors of proteins in the cell can be observed, or phosphorylation targets can be searched comprehensively.

In the antibody array, different kinds of antibodies are arranged and immobilized on a support. The antibody array is also known as an antibody chip. By allowing the antibody array to bind to proteins, proteins that interact with the target antibodies can be detected.

In the enzyme array, different kinds of enzymes are arranged and immobilized on a support. The enzyme array is used for the purpose of monitoring activities of different kinds of enzymes, for example.

In the SH3 domain array, a group of proteins with an SH3 region are arranged and immobilized on a support. A representative example is the array manufactured by Panomics.

The SH3 (Src Homology 3) domain is a relatively short conserved region that occurs in Src protein. Specifically, the SH3 domain has a beta-barrel structure of 50 to 70 amino acid residues with five to six anti-parallel beta strands packed together. The SH3 domain specifically binds to a target protein via a peptide region (SH3 ligand) with a common sequence of six to twelve residues. Two types of SH3 ligands are known, both of which contain prolines. The binding is made as the proline occupies a hydrophilic pocket.

In humans, about 408 kinds of proteins with SH3 domains are known. These proteins serve as mediators of various interactions, playing part in cell-cell communications, or signal transduction from a cell surface to the nucleus. Thus, with the SH3 domain array, it is possible to recognize involvement of a specific protein in a particular signal transduction, or the number of proteins involved in a specific reaction pathway.

In the receptor array, receptor proteins associated with various cell responses are arranged and immobilized on a support. The receptors are not particularly limited and not necessarily limited to proteins as long as they can specifically recognize substances such as hormones, neurotransmitters, or foreign substances such as autacoid, or respond to physical or chemical stimuli, when these substances or stimuli induce cell response. Generally, the receptors are proteins which are activated by specific substances or stimuli present in the cell membrane, organelle membrane, or cytoplasm.

Some of the arrays described above are classified as so-called biological chips, which are particular type of protein chips.

The protein chip is a small array (chip) on which various chemical properties suitable for protein analysis are spotted and immobilized. Depending on the purpose of analysis, the protein chip is broadly classified as a chemical chip and a biological chip. The biological chip is used for the analysis of specific binding (interaction) of proteins or other polypeptides. As the substances immobilized on the chip, substances, for example, such as antibody, receptor, or DNA are used that can interact with polypeptides. Thus, in terms of purpose (use), the nucleic acid-immobilized array described in the First Embodiment can also be classified as a biological chip. As to the chemical chip, details will be described later.

In some types of biological chips, a carbonyldiimidazole group or epoxy group is immobilized on the surface. These functional groups (compounds) can easily immobilize biosubstances such as an antibody, receptor protein, or DNA, allowing an array to be easily fabricated according to the purpose of analysis. In other words, an array according to the present invention may be adapted so that biosubstances (or synthetic substances) are immobilized either directly on a surface of a support, or, as in the biological chip, with an intervening ligand compound that can desirably bind to the support surface and the biosubstances.

### [Third Embodiment]

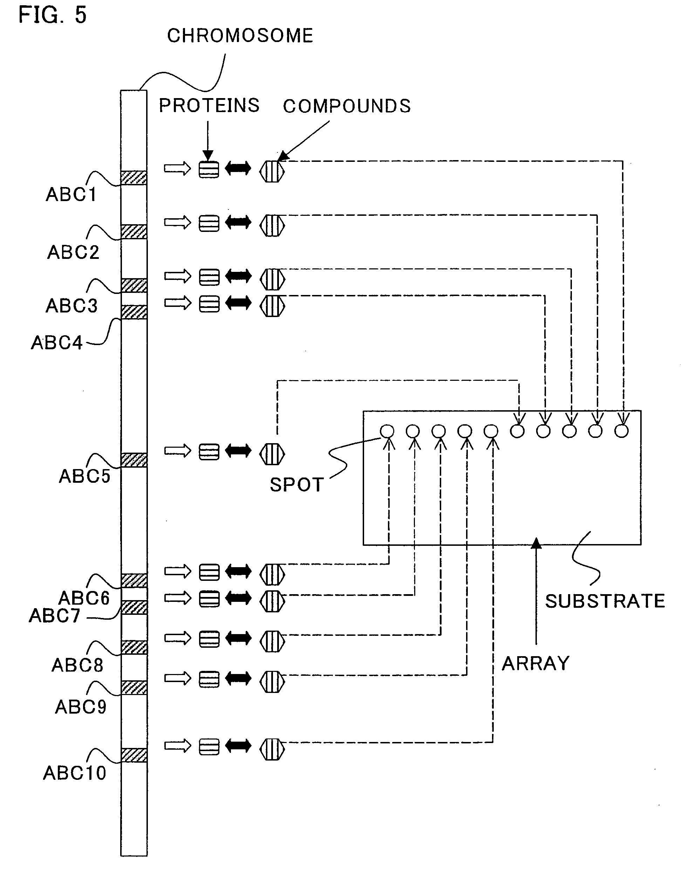

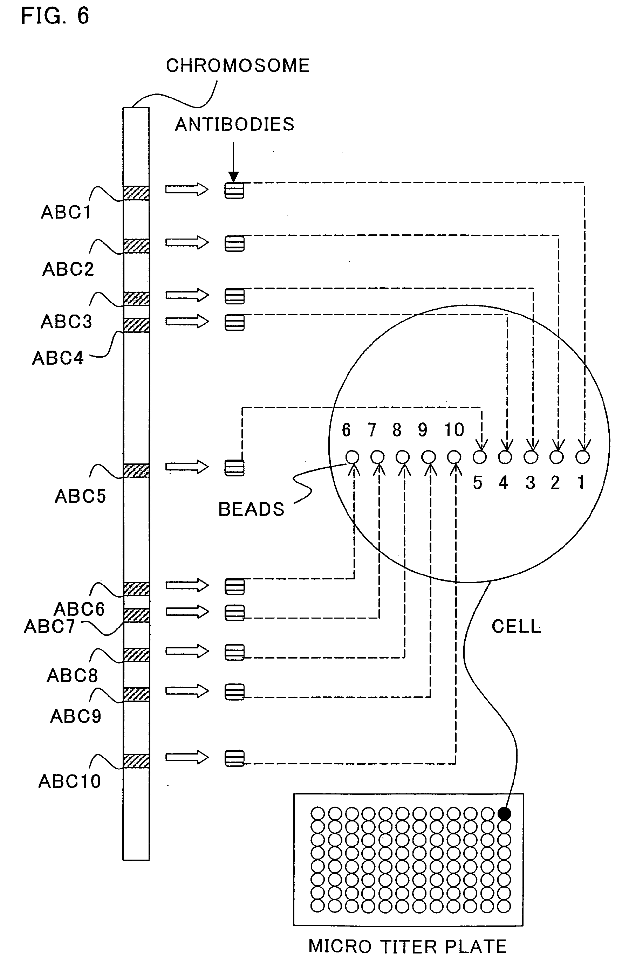

Referring to Fig. 5 and Fig. 6, the following will describe yet another embodiment of an array according to the present invention. It should be appreciated that the invention is not limited by the following description.

In the foregoing First and Second Embodiments, the present invention was described through the case where nucleic acids and polypeptides are used as biosubstances, respectively. However, the invention is not limited to these examples, and synthetic substances that can interact with the biosubstances can also be used.

### <Examples of synthetic substances>

The synthetic substance is not particularly limited as long as it can interact with the biosubstance. Specific examples are compounds with a protein-interacting group, which may be a hydrophobic group, cation-exchange group, anion-exchange group, metal ion immobilized group, or normal phase group. The synthetic substances also include synthetic oligonucleotides and synthetic oligopeptides.

In an array of the present embodiment, as in the First and Second Embodiments, the synthetic substances immobilized on a support are arranged in the order respective base sequence blocks of the biosubstances interacting with the synthetic substances are sequenced on the chromosome.

For example, as in Fig. 1 and Fig. 3, it is assumed here that an array is fabricated for an organism Z based on an organism Z chromosome in which 10 genes ABC1 through ABC 10 are present that are lined up in this order on the chromosome, as schematically illustrated in Fig. 5. It is also assumed that 10 kinds of proteins are respectively transcribed and translated from the genes ABC1 through ABC10 (as indicated by arrows), and that the proteins specifically interact with a certain compound. In this case, an array is fabricated by spotting the compound in the order genes encoding the proteins interacting with the compound are sequenced on the chromosome.

A specific example of an array using the synthetic substances is a chemical chip as one type of the protein chip described in the foregoing embodiment. The chemical chip is generally used for the expression analysis, purification, and identification of proteins, whereas the biological chip is used for the evaluation of specific binding (interaction) of the proteins, for example.

As described above, the protein chips include chemical chip and biological chip. In the chemical chip, a functional group (compound) such as a hydrophobic group, cation-exchange group, anion-exchange group, metal ion immobilized group, or normal phase group is immobilized on a chip surface. As with common chromatography, the chemical chip is used such that, when brought into contact with a sample under certain reaction conditions, the functional group can capture the proteins in the sample. The sample is not particularly limited as long as it contains (or may contain) proteins. Specific examples include biological samples such as serum, urine, spinal fluid, synovial fluid, saliva, and tissue homogenate; and culture samples such as cultured-cell supernatant or cultured-cell crushed solution.

### <Protein chip system>

The method of analyzing the protein chip, including both the chemical chip and the biological chips described in the Second Embodiment, is not particularly limited. Generally, a protein chip system is used. The protein chip system is not limited to a particular structure, and is generally realized by a computer including: a protein chip, a protein chip reader used for measurement, and software for measurement and analysis. The protein chip system may also include other components as well.

The protein chip reader is not particularly limited as long as it can read out data of expression analysis or interaction evaluation of proteins from the protein chip. Generally, a Time-of-Flight Mass Spectrometry (TOS-MS) is used for this purpose. In the TOS-MS, an ionized sample is allowed to fly through a highly evacuated column by applying kinetic energy of a constant acceleration voltage. The time of flight of the sample reaching a detector is then measured to analyze the mass of the sample. In this way, data of expression analysis or interaction evaluation of proteins or the like is read out from the protein chip.

The sample used in the TOS-MS may be polypeptides such as proteins, or nucleic acids such as DNA. The method of ionizing the biosubstance sample is not particularly limited, and generally, a MALDI (Matrix Assisted Laser Desorption/Ionization) method is used. In this method, a sample immobilized on a metal plate (support) is ionized by irradiation of a laser beam. The TOS-MS using the MALDI method is called MALDI-TOF-MS.

The following describes an exemplary analysis method using the protein chip system. First, one to several hundred micro liters of sample is spotted on a protein chip. Then, the surface of the protein chip is washed under predetermined conditions, so as to remove substances that do not interact with the substances immobilized on the protein chip surface. In this way, proteins captured on the spot under specific conditions are selected. Each spot is then ionized by MALDI, and the molecular weight is measured by TOS-MS. The data obtained from each spot is analyzed by a computer.

The protein chip system allows a large number of samples to be analyzed both quickly and quantitatively from a small amount of sample and based on the mass number, without using any label or tag. Further, the system allows for measurement of a trace component in a crude sample without pre-treatment. Further, residual salts on the spots can be easily removed before measurement is performed. The system is therefore suitable for the search of marker proteins of various diseases, or evaluation of toxicity, or for screening molecules (candidate substances for drugs) that interact with specific molecules.

Here, the advantages described in the First Embodiment can be obtained if the synthetic substances or biosubstancs immobilized on the protein chip are arranged in the order they are coded on the chromosome. In this case, the protein chip system can improve the level of analysis it performs, or can be used in more practical applications.

### <Bead array system>

In order to efficiently identify proteins, the bead array as described in the First Embodiment can be used. In the bead array, as shown in Fig. 6, a plurality of beads (10 beads in Fig. 6) respectively appended with ID codes are charged in a small container formed by a cell of a micro titer plate. On the surface of each bead, a probe such as a biosubstance or synthetic substance (antibody in this example) is immobilized.

With the sequence position information appended to the beads, the type of protein (which of the 10 proteins transcribed and translated (as indicated by the arrows) from the genes ABC1 through ABC 10 as shown in Fig. 6) corresponding to the probe immobilized on the surface can be specified. With a two-wavelength laser beam, 100 kinds of beads may be quantified.

This technique can be used for detection in a liquid phase, and therefore is useful for quantification of proteins in particular. A representative example of the bead array system is the fluorescent micro bead array system Luminex, the product of Hitachi Software Engineering Co., Ltd.

The bead array system is not limited to a particular structure, and is generally realized by a structure including: a plate with probes, an analyzer used for fluorescence detection, and a computer equipped with software for measurement and analysis. The micro bead array may include other elements as well.

The analyzer is not particularly limited as long as it can read out the result of expression analysis or interaction evaluation of the proteins, in some form of data, from the bead array. Generally, a device equipped with a flowmetry mechanism and fluorescence detection capability using a laser beam can be used. The device can distinguish tones of bead colors. Thus, by immobilizing different antibodies on the beads and allowing the antibodies to bind to labeled samples, the level of sample binding can be measured for each bead by flowcytometry. The samples can then be quantified from these reactions by gathering several hundred samples for each type of bead.

The sample used in the bead array system may be polypeptides such as proteins, or nucleic acids such as DNA. The bead array system allows a large number of samples to be analyzed in a liquid phase both quickly and quantitatively from a small amount of sample. Here, the advantages described in the First Embodiment can be obtained if the synthetic substances or biosubstances immobilized on the bead array are arranged in the order they are coded on the chromosome.

It should be appreciated here that the invention is not just limited to the foregoing embodiments and various modifications are possible within the scope of the invention as defined in the appended claims. Embodiments obtained by suitably combining different technical means as disclosed in the embodiments also fall within the scope of the present invention. Thus, even though the foregoing Third Embodiment was described through the case of bead array and protein chip as arrays according to the present invention, an array analyzing system such as the protein chip system is also applicable, for example, to the DNA micro array described in the First Embodiment.

As described above, in an array according to the present invention, biosubstances, or synthetic substances that interact with the biosubstances, are analyzed by arranging these substances in the chromosomal order of the genes that encode the biosubstances. This enables the array to be used in more practical applications such as screening in variety improvement, in addition to improving reliability of array analysis.

Note that, an array according to the present invention may be provided as a kit according to intended use. For example, in the case where the DNA array described in the First Embodiment is used for variety improvement, the array may be provided as a kit including reagents or instruments for preparing target DNA.

### [Fourth Embodiment]

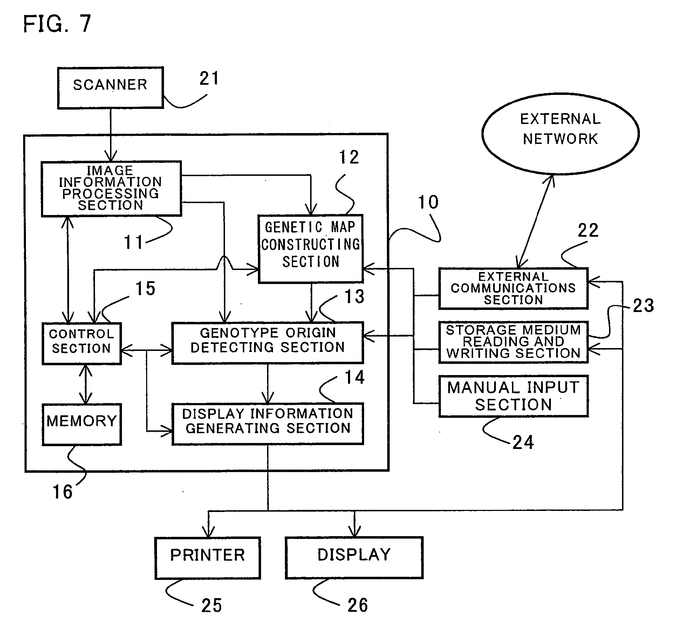

Referring to Fig. 7 through Fig. 9, the following will describe one embodiment of a genotype analyzing and display system according to the present invention. It should be appreciated that the invention is not limited by the following description.

In a genotype analyzing and display system according to the present invention, analysis is made for hybrid individuals derived from the cross between individual A and individual B (A 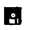 B) of an arbitrarily selected species of living organism, using the result of hybridization performed with the nucleic acid array. From the result of analysis, the system provides a graphical representation of locations of the chromosomes of the hybrid individuals where crossovers have occurred.

A genotype analyzing and display system according to the present invention is not limited to a particular structure. Specifically, as shown in Fig. 7, a genotype analyzing and display system includes, for example, an image information processing section (image information processing means) 11, a genetic map constructing section (genetic map constructing means) 12, a genotype origin detecting section (genotype origin detecting means) 13, a display information constructing section (display information constructing means) 14, a control section (control means) 15, a memory (storage means) 16, a scanner (image reading means, input means) 21, an external communications section (external information input and output means) 22, a storage medium reading and writing section (memory means, input means, output means) 23, a manual input section (manual input means) 24, a printer (image forming means, printing means, output means) 25, and a display (image display means, output means) 26. The genotype analyzing and display system of such a structure can be roughly divided into an input section, an output section, and an analyzing section (analyzing means) 10.

### (I) Nucleic acid array

### <Specific structure of nucleic acid array>

The invention analyzes and displays a genotype of a desired species of living organism based on the result of analysis performed with a nucleic acid array on the expression level of genes of hybrid individuals derived from the cross between individual A and individual B (A 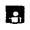 B). The nucleic acid array used in the present invention is not particularly limited, and conventional nucleic acid arrays can be suitably used. Specific examples include a micro array, a macro array, and a bead array. In the present embodiment, DNA is used as the nucleic acid, and therefore more specific examples of the nucleic acid array are DNA arrays such as a DNA micro array and a DNA macro array.

The DNA micro array is also known as a DNA chip, and the immobilized DNA is often referred to as a probe. The micro array is smaller in size than macro array and provides more density. This enables the number of genes (DNA fragments) immobilized as probes to be increased, allowing for more comprehensive gene expression analysis.

The DNA micro array can be classified based on types of immobilized DNA. However, structural differences can be revealed more clearly if the DNA micro array is classified based on fabrication methods. Specifically, based on fabrication methods, the micro array can be broadly classified into the Stanford type and the Affymetrix type.

A DNA micro array of the Stanford type is fabricated by spotting a DNA solution onto a substrate (support) with a spotter, wherein a slide glass for a microscope is used as the substrate. One advantage of a DNA micro array of a Stanford type is that it can always be fabricated with the use of a spotter. However, this comes with a drawback in that it requires expensive hardware (spotter, etc.), or complex procedures for the preparation of biosubstances as necessitated by a large number of probes required for spotting.

On the other hand, a DNA micro array of the Affymetrix type does not employ the method of immobilizing DNA fragments on a substrate with a spotter, etc., but is fabricated by chemically synthesizing oligoDNA of about 25 mer on a substrate using a micro fabrication technique commonly used in the fabrication of semiconductors, namely, a photolithography technique.

Specifically, for each gene, 11 to 20 oligos (25 mers) (for example, 11 oligos in the case of a barley DNA array) are set based on base sequence data, and a pair of oligo DNA: one with a perfect match to each 25 mer, and one with a forced single-base mismatch at the 13th base is used as a probe. The array can be fabricated without using a spotter or other devices when it is designed with data of a known database. Further, since the probe (DNA fragment) has a constant length and the sequence is known, the GC content, which influences the strength of hybridization, can remain constant. It should be noted, however, that since the probe is synthesized based on information of a database, clones to be analyzed need to be separately isolated.

In the present invention, either the Stanford DNA micro array or the Affymetrix DNA micro array can be used as the nucleic acid array.

The following describes an exemplary method of using the DNA micro array. First, the DNA micro array is hybridized with fluorescent-labeled target DNA (hereinafter, "targets"). Here, the target molecules containing complementary sequences to the probes on the DNA micro array bind to (hybridize with) their complementary probe molecules, leaving other target molecules unbound. Then, these target molecules not bound to the probes are washed and removed, leaving only the hybridized target molecules on the micro array. Since the target molecules are fluorescence-labeled, the fluorescence of the targets is measured as signal intensity and hybridized probes are identified.

The fluorescent-labeled targets are generally prepared first by extracting mRNA from cells of two different states (first state and second state) to be compared, and then performing a reverse transcription reaction in the presence of fluorescent nucleotides. Here, two kinds of fluorescent dyes with different detection wavelengths are used for the first state and second state, respectively. The expression level of genes is greater for the cDNA contained in the targets, and the fluorescent signal intensity is in accord with the expression level of genes in each state. Thus, from the measured signal intensity, the expression level of a specific gene can be detected.

The DNA macro array basically has the same structure as the DNA micro array, but differs from the DNA micro array in that it uses a common membrane filter like a nylon membrane. An advantage of the macro array is that it allows for an expression profile analysis, genome wide, according to methods based on conventional blotting methods. Another advantage is that, unlike the micro array, the DNA does not detach in washing, owning to the fact that the spotted DNA is immobilized on a membrane filter after denatured by an alkali treatment. Therefore, the macro array and micro array should be suitably selected according to use.

The following describes an exemplary method of using the macro array. The macro array is used basically in the same way as the micro array. Specifically, the macro array is hybridized with isotope (³³P, etc.)-labeled targets. Then, target molecules that did not bind to the array are washed and removed, leaving only hybridized target molecules on the macro array. Here, since the target molecules are isotope-labeled, the spots are exposed on an imaging plate and the expression level of the targets is determined by measuring signal intensity from the imaging plate--a procedure not performed in the micro array.

The techniques of the present invention can also be applied to the mass array. In the mass array, genomic DNA fragments are arranged and immobilized in an orderly manner on a silicon substrate, and therefore the structure is basically the same as that of the micro array. The mass array was developed for SNP analysis, and as such it is used differently from the DNA micro array.

Specifically, oligonucleotides corresponding to regions in the vicinity of target SNP are synthesized and hybridized with the mass array. Then, by using the oligonucleotides as primers, a DNA fragment having a SNP single base difference is synthesized through elongation catalyzed by DNA polymerase. The DNA fragment is eluted and then ionized with MALDI. The SNP type can be determined by detecting a single base mass difference using TOS-MS.

The bead array can also be used as the nucleic acid array of the present invention. The bead array is used in such a manner that, in a small container, a probe such as a nucleic acid or antibody is immobilized on a surface of each bead to which an ID code has been added, and that the probe immobilized on the probe surface is specified by reading the ID code of the bead. With use of a two-wavelength laser beam, 100 kinds of beads can be quantified. That is, in an array according to the present invention, the support may be a collection of micro arrays (beads, for example) on which biosubstances or synthetic substances are individually immobilized.

### <Chromosomal location recognizable array>

The nucleic acid array used in the present invention is most preferably the chromosomal location recognizable array described in the First Embodiment. In the chromosomal location recognizable array, the probes (a plurality of nucleic acid molecules) immobilized on a support are arranged in such an orderly manner that the chromosomal order of respective base sequence blocks of the nucleic acid molecules is recognizable. In the most typical chromosomal location recognizable array, the nucleic acid molecules (probes) are arranged in the chromosomal order of respective sequence blocks of the base sequences of the probes. That is, the probes are arranged in the chromosomal order. With the probes arranged in the chromosomal order, the chromosomal location recognizable array provided as a nucleic acid array is usable as a micro array, a macro array, a mass array, and the like.

In the bead array, the beads immobilizing the probes are appended with sequence position information indicative of the chromosomal order of base sequence blocks corresponding to the base sequences of the probes. In use, the analysis result (result of hybridization) is acquired and the sequence position information is read out, so that the order obtained from the analysis result is rearranged in the chromosomal order.

The nucleic acid molecules immobilized on the nucleic acid array may be DNA or RNA, and DNA is generally used as described above. The type of DNA used as the probes immobilized on the DNA array (nucleic acid array) is not particularly limited, but a genetic marker, genomic DNA, genomic DNA treated with restriction enzyme, cDNA, EST, and synthetic oligoDNA are preferably used, for example. It is preferable that the DNA be arranged based on a genetic map or physical map. For example, for a group of different kinds of genetic markers, it is preferable that these genetic markers make up a genetic map. Based on the genetic map, the DNA fragments can be arranged on a substrate.

The genetic marker or a group of genetic markers are not particularly limited as long as they can serve as genetic labels on the chromosome. Non-limiting examples include an EST marker using EST, a SNP marker including SNP (Single Nucleotide Polymorphism), a RFLP (Restriction Fragment Length Polymorphism) marker, and a micro satellite marker (SSR (simple sequence repeat) marker). Thus, the genetic marker or a group of genetic markers include genomic DNA treated with restriction enzyme, EST, synthetic oligoDNA, and the like, if they can be used as markers.

The number of DNA (probes) immobilized on the support is not particularly limited, and it is generally on the order of several thousand (10³). The number of immobilized (or arranged) DNA varies greatly depending on the type of device, such as a spotter, used for the fabrication of the array, or the area of the support (substrate), for example.

It should be noted that, in the DNA array, information concerning gene expression can only be obtained for genes corresponding to the immobilized DNA fragments. It is therefore preferable to increase the number of immobilized DNA (probes) as much as possible, in order to perform gene expression analysis more systematically and comprehensively.

### (II) Structure of genotype analyzing and display system

### <Input section>

The invention analyzes and displays a genotype of a desired species of living organism based on the result of analysis performed with an nucleic acid array on the expression level of genes of hybrid individuals derived from the cross between individual A and individual B (A 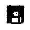 B). To this end, a genotype analyzing and display system according to the present invention includes means (input section) for inputting the result of hybridization analysis performed with a nucleic acid array on the expression level of genes, i.e., comprehensive information concerning expression level of genes of the hybrid individuals being analyzed.

In the structure shown in Fig. 7, the analysis result from the nucleic acid array is entered as image information through the scanner 21. The image information processing section 11 analyzes the image information and generates expression level information of genes from the analysis result. The scanner 21 is not particularly limited as long as it can serve as image reading means for reading the hybridization result as image information. Specifically, the fluorescence of the targets that hybridized with the probes is read out as image data from the nucleic acid array, and the expression level of genes is detected from the signal intensity of the image data. Thus, as the scanner 21, a conventional fluorescent scanner 21 can be suitably used, for example.

Here, the image information obtained from the scanner 21 is subjected to necessary information processing to generate gene-expression-level information. Thus, as shown in Fig. 7, the present invention preferably includes the image information processing section 11 for analyzing the expression level of genes based on the image information, and generating comprehensive gene-expression-level information. The image information processing section 11 is not limited to a particular structure, and conventional gene expression analyzing systems can be used.

Further, in the present invention, the result of hybridization analysis from the nucleic acid array (gene-expression-level information of the hybrid individuals) is compared with the genetic information of the parents of the hybrid individuals, and with the genetic map of the species to which these individuals belong, as will be described later. To this end, a genotype analyzing and display system according to the present invention includes, in addition to the scanner 21 (image reading means), means for inputting genetic information of the parents, and information of the genetic map.

The means for inputting genetic information of the parents is not particularly limited. In the structure shown in Fig. 7, the external communications section 22, the storage medium reading and writing section 23, and the manual input section 24 correspond to such means. Generally, the genetic information of individuals of the parental generation is well known, and therefore genetic information of the parents may be obtained from database via networks with the external communications section 22, or genetic information stored in various storage media may be read out with the storage medium reading and writing section 23. Further, if the information can be manually entered, it may be entered through the manual input section 24.

The external communications section 22 is not particularly limited as long as it allows for input and output of information to and from external devices, and conventional communications interfaces such as a LAN card, a LAN board, a LAN adapter, and a modem can be used. The storage medium reading and writing section 23 is not limited to a particular structure either. For example, known disk drives such as a hard disk drive, a flexible disk drive, a CD-ROM drive, and a DVD-ROM drive, or various memory cards or memory cartridges such as USB memory can be used. The manual input section 24 is not limited to a particular structure, and conventional input means such as a keyboard or a tablet can be suitably used.

The genetic information of parents is not particularly limited, and may be genotypes of parents, or gene expression profile information, for example. Among these examples, genotype information of parents is more likely to be known. In particular, genotypes of organisms used for experiment, or genotypes of important species in crops, domestic animals, or the like are widely known and some are available as a database. Thus, genotype information can be suitably used as genetic information of parents.

The gene expression profile information is obtained through a comprehensive analysis of gene expression in the cell. Under ordinary or specific conditions, the expression pattern of genes may vary depending on the genotypes of individuals. Thus, the gene expression profile information can be used as genetic information of parents, instead of the genotype information. Further, the gene expression profile information may be used together with the genotype information, so as to enhance genetic information of parents.

The means for entering genetic map information is not particularly limited, and the means for entering genetic information of parents may be used therefor. This is because a relatively large number of genetic maps are available as complete chromosome maps, as with the genetic information of parents. However, given that fact that sufficient genetic maps are not available for the majority of agricultural crops or domestic animals, the invention may include, for example, the genetic map constructing, section 12 for constructing a necessary genetic map based on genetic map constructing information, as shown in Fig. 7. The genetic map constructing section 12 will be described in more detail later.

Note that, the comprehensive gene-expression-level information of the hybrid individuals may be entered without being mediated by the scanner 21 or image information constructing section 11. For example, gene-expression-level information having been entered and analyzed through the scanner 21 or other gene expression analyzing systems may be entered through the external communications section 22 or storage medium reading and writing section 23.

Further, it is preferable that a genotype analyzing and display system according to the present invention include means for correcting at least one of the comprehensive gene-expression-level information of hybrid individuals, genetic information of parents, and genetic map constructing information. Specifically, the manual input section 24 shown in Fig. 7 corresponds to such means.

As will be described later, a genotype analyzing and display system according to the present invention performs an analyzing steps in which an entry error is found, if any, when creating a genetic map or display information. In this way, reliability of final display information can be improved. It is therefore preferable that means be provided for correcting an entry error. Specifically, the manual input section 24 can be used to for this purpose. The means for correcting an entry error is not just limited to the manual input section 24, and other means may be used as well.

### <Analyzing section>

The present invention analyzes and displays genotypes of hybrid individuals of the cross between individual A and individual B (A 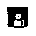 B) in a desired species of living organism, using the result of analysis performed on the expression level of genes with a nucleic acid array. Thus, the analyzing section 10 for analyzing the information entered through the input section is an essential component. The analyzing section 10 at least includes the genotype origin detecting section 13 and the display information generating section 14, as shown in Fig. 7.

In the genotype origin detecting section 13, the gene-expression-level information or polymorphism information generated in the image information processing section 11 through the scanner 21, or the gene-expression-level information entered through the external communications section 22 or the storage medium reading and writing section 23, etc., is compared with the genetic information of parents or the genetic map, so as to determine whether the genotype of a hybrid individual of interest derived from which parent. This is not limited to a particular process, and the genotype origin detecting section 13 can suitably perform the process according to the selected procedure of crossing, or the type of species of living organisms used. For example, a genotype may be determined as being signal parental, hetero, or unrecognizable to yield the result.

The determination is made using the genetic information of the parents (genotype information, expression profile information) and a genetic map, by comparing these information with the gene-expression-level information and polymorphism information. The polymorphism information may be of SNP or RFLP, for example. The polymorphism information is generally used as a genetic marker, and therefore the presence or absence of a genetic marker distinct to the genotype being compared may be used as a criterion of the determination. It should be appreciated however that the present invention is not just limited to this example, and the polymorphism information is not necessarily required in the comparison as long as the determination yields effective results.

The display information generating section 14 gathers the results of determination obtained in the genotype origin detecting section 13, and displays genotypes as a whole on the chromosome basis based on the results of determination, so that the parental type of the genotypes can be individually recognized. The display information is not particularly limited as long as the origin of each genotype can be recognized on the chromosome basis. For example, it is preferable that the display information be generated with statistics. The statistics included in the display information is not particularly limited, and may be, for example, at least one of, or preferably both of a recombination number and recombination frequency of individual chromosomes. With such statistics included in the display information, information concerning crossovers can be recognized comprehensively for each chromosome, in addition to recognizing the origin of individual genotypes.

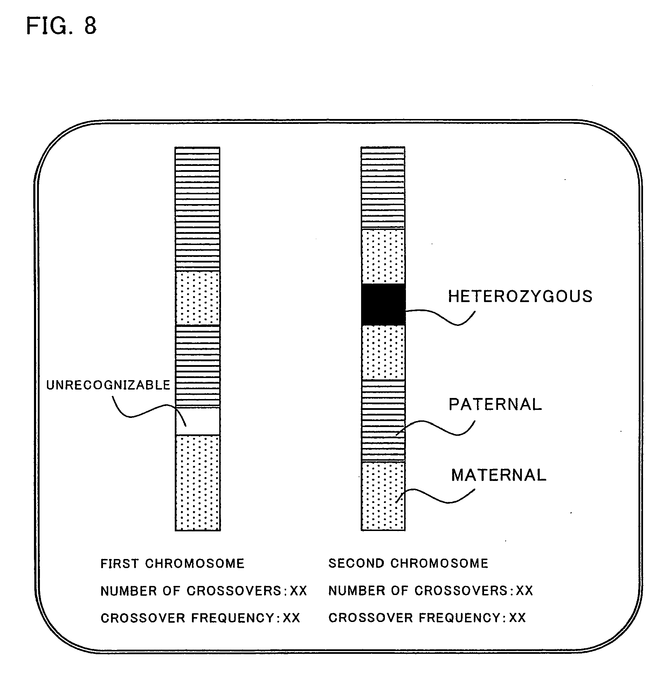

The display information is adapted so that the origin of each genotype can be recognized on the chromosome basis. Specifically, as shown in Fig. 8, the origin of a genotype of interest can be recognized by different display colors or display patterns. In the example illustrated in Fig. 8, the stripe region indicates paternal origin (one of the parents), the dotted region indicates maternal origin (the other parent), and solid region indicate hetero origin. The blank region means that the origin is unrecognizable. The display is not limited to such display patterns, and different colors may be displayed to make distinctions.

A map of genotypes of different individuals of a given species of living organism is generally referred to as a graphical genotype. From the graphical genotype, whether a particular individual includes a particular trait (locus) can be found if a marker linked to the trait is available. Thus, the present invention can be thought of as a technique of displaying genotypes of parents as graphical genotypes, based on the result of hybridization analysis performed on the expression level of genes with a nucleic acid array. As such, for the display information generated in the present invention, a display method used in the graphical genotype can be suitably used.

A genotype analyzing and display system according to the present invention may include the genetic map constructing section 12, in addition to the genotype origin detecting section 13 and the display information generating section 14. The genetic map constructing section 12 constructs a genetic map of a species to which the hybrid individuals belong, based on genetic map constructing information. As described earlier, the genetic map is constructed for only some of the species. It is therefore preferable to provide the genetic map constructing section 12.

The genetic map constructing section 12 is not particularly limited as long as the genetic map is constructed on the chromosome basis based on various genetic map constructing information. As the genetic map constructing information, at least names of genes and/or genetic markers known in the species being analyzed, and the chromosomal loci of the genes and/or genetic markers are used, for example.

The means for entering the genetic map constructing information is not particularly limited, and various input sections, for example, such as the external communications section 22, the storage medium reading and writing section 23, and the manual input section 24 shown in Fig. 7 can be used.

Further, with the chromosomal location recognizable array, a genetic map can be constructed through mapping of genetic markers with unknown locations. Specifically, in order to construct a genetic map, targets obtained from Mendelian segregation population of the species being analyzed are hybridized with the chromosomal location recognizable array. Then, genetic markers with unknown locations are hybridized on the same chromosomal location recognizable array, so as to determine locations of the genetic markers. In this way, a high density genetic map can be constructed.

Even though the foregoing example uses the same chromosomal location recognizable array, the method of mapping the genetic markers of unknown location is not just limited to this example. For example, mapping can be made by processing the same targets with the genetic markers on different arrays. Here, mapping of genes is possible if the genes follow the rule of Mendelian segregation as in Single Feature Polymorphism (SFP), even if SNP or RFLP is not detected.

Thus, a genotype analyzing and display system according to the present invention may be adapted so that, in order to construct a genetic map in the genetic map constructing section 12, the hybridization result is analyzed and processed by reading it from the array with the scanner 21 and the image information processing section 11, before analyzing genotypes. To this end, the image information processing section 11 is adapted to output information also to the genetic map constructing section 12, as shown in Fig. 7 (as indicated by arrow in the figure).

Information such as the genetic map constructed by the genetic map constructing section 12, or the result of determination made by the genotype origin detecting section 13 can be temporarily stored in the memory 16. The memory 16 is provided in the analyzing section 10 as shown in Fig. 7, and serves as a storage section for storing various information used or generated in a genotype analyzing and display system according to the present invention. The memory operation of the memory 16 is controlled by the control section 15. The memory 16 is not limited to a particular structure, and may be realized, for example, by a semiconductor memory, such as RAM or ROM. Note that, the storage medium reading and writing section 23 described as an input section can be used as a storage section of the present invention. This will be described later in more detail in conjunction with the output section.

The analyzing section of the structure shown in Fig. 7 includes the control section 15 for controlling the entire operation of the analyzing section 10, and in turn the entire operation of the genotype analyzing and display system. In the structure shown in Fig. 7, the control section 15 outputs control information to the image information processing section 11, the genetic map constructing section 12, the genotype origin detecting section 13, the display information generating section 14, and the memory 16. These means operate based on the control information they receive, thereby operating the genotype analyzing and display system. It should be noted here that the control section 15 is also adapted to receive information from these means, and as such the flow of control information is indicated by the bidirectional arrow in Fig. 7.

### <Output section>

The present invention analyzes and displays genotypes of hybrid individuals of the cross between individual A and individual B (A 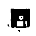 B) in a desired species of living organism, using the result of analysis performed on the expression level of genes with a nucleic acid array. To this end, a genotype analyzing and display system according to the present invention includes means, provided as an output section, for outputting display information.

The output section for outputting the display information is not particularly limited, and at least one of, or preferably both of a display 26 for displaying display information on a display screen (soft copy), and a printer 25 for printing display information (hard copy) are provided. The display 26 is not limited to a particular structure, and various types of known displays such as a CRT, a liquid crystal display, and a plasma display can be used. The printer 25 is not limited to a particular structure, and known image forming devices such as an ink-jet printer and a laser printer can be used.

It is preferable that the display 26 and the printer 25 are both adapted to output display information in colors. This enables the origin of a genotype to be displayed in different colors, thereby increasing display variations. Use of color is also preferable in displaying the origin of a genotype in the display patterns shown in Fig. 8, because colors offer graphical representations that are easier to read.

The output section is not just limited to the display 26 or printer 25, and other means can be used as well. For example, the external communications section 22 can be used as an output section. Specifically, the external communications section 22 allows for input and output of information to and from external devices, by serving as both an input section and an output section. This enables display information to be transmitted to other devices via external networks, etc, enabling a genotype analyzing and display system according to the present invention to be used more efficiently.

Specifically, when the genotype analyzing and display system is connected to external devices via LAN for example, the genotype analyzing and display system, installed in a research facility for example, can be shared with other researchers via information terminals such as personal computers. Further, the results of analysis obtained in the genotype analyzing and display system may be accumulated in an external server via a communications network, allowing the analysis result to be used more efficiently.

As the output section, the storage medium reading and writing section 23, described as an input section, can be suitably used. Specifically, in a genotype analyzing and display system according to the present invention, a drive for reading information from a storage medium can be used as an output section if the drive has a writing capability. The storage medium reading and writing section 23 is not limited to a particular structure, and known disk drives such as a hard disk drive, a flexible disk drive, a CD-ROM drive, and a DVD-ROM drive, or various memory cards or memory cartridges such as a USB memory can be suitably used for example, as described above in conjunction with the input section.

Note that, in the exemplary structure shown in Fig. 7, the system is realized by the analyzing section 10 and independently provided input and output sections, wherein the analyzing section 10 includes the image information processing section 11, the genetic map constructing section 12, the genotype origin detecting section 13, the display information generating section 14, the control section 15, and the memory 16. However, the present invention is not just limited to this structure. For example, all means may be provided as a single unit, or some of the input sections and/or output sections may be integrated with the analyzing section 10. Further, the system may include means other than those shown in Fig. 7.

The analyzing section 10 is not just limited to a particular structure, and conventional arithmetic means, for example, such as a central processing unit (CPU) of a computer may be used. The operation of the analyzing section 10 is executed by a computer program.

### (III) Analyzing method by the genotype analyzing and display system

An analyzing method performed by a genotype analyzing and display system according to the present invention is not particularly limited. Specifically, the method may include 12 steps as shown in Fig. 8.

First, in step 101 (step will be denoted by "S" hereinafter), genetic map constructing information (names of chromosomes, genes, and genetic markers, and loci, etc.) is entered through input sections. In S102, the genetic map constructing section 12 constructs a genetic map based on the genetic map constructing information, and the genetic map is supplied to the genotype origin detecting section 13. Here, the genetic map may be stored in the memory 16, or optionally displayed in the display 26. In S103, the presence or absence of an entry error is found (need for correction is determined). If there is an entry error (YES), the genetic map constructing information is re-entered in S104 through, for example, the manual input section 24, and the sequence returns to S101.

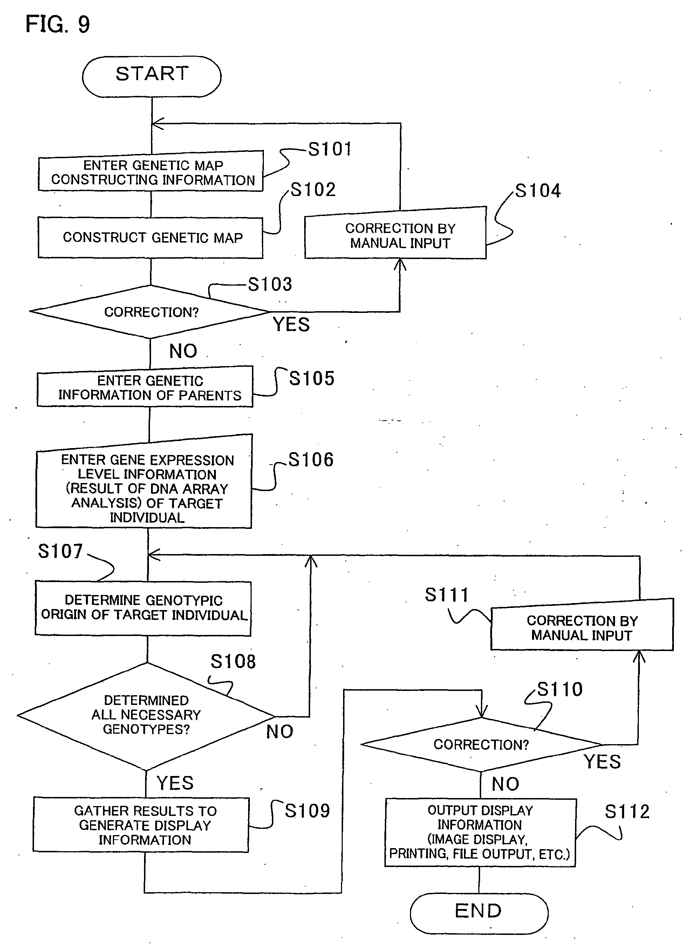

On the other hand, if correction is not required (NO), the sequence goes to S 105. In S105, genetic information of parents (genotypes of parents and/or gene expression profile information) is entered through input sections. In S106, gene-expression-level information (DNA array analysis result) of hybrid individuals (target individuals in Fig. 9) being analyzed is entered through the scanner 21 and image information processing section 11. In S 107, the genotype origin detecting section 13 compares the comprehensive gene-expression-level information of the hybrid individuals with the genetic information of the parents, and the genetic map of the species to which these individuals belong, so as to determine whether a genotype of a hybrid individual of interest derived from which parent. In S108, it is decided whether the determination has been made for all necessary genotypes. If not (NO in Fig. 9), the sequence returns to S107 and the procedure is repeated.

On the other hand, if the determination has been finished for all genotypes in S108, the display information generating section 14 in S109 gathers results of determination and, based on the results, generates display information, the display information being generated for displaying a plurality of genotypes together on the chromosome basis so that the parental type of individual genotypes can be recognized. Here, the display information may be stored in the memory 16, or optionally displayed in the display 26. Then, in S110, the presence or absence of an error in generating the display information is found (need for correction is determined). If there is an error (YES), correction information is entered in S111 through the manual input section 24, and the sequence returns to S107. If correction is not required (NO), the output section outputs the display information in S112. This completes the series of analysis procedures.

### (IV) Use of the present invention

Use of a genotype analyzing and display system according to the present invention is not particularly limited, and the system is used for graphically displaying locations of crossovers that have occurred on the chromosomes of hybrid individuals of the cross between individual A and individual B (A 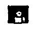 B) of an arbitrary species of living organism, based on the result of analysis using the hybridization results obtained with the use of a nucleic acid array.

As a specific example, the invention can be suitably used for identifying a target trait-containing chromosome fragment from hybrids of organisms (identifying a genotype), or selecting a variety with a target trait from hybrids of organisms being crossed for variety improvement. With the present invention, the site of recombination on the chromosomes can easily be found. This allows for accurate selection of only those individuals with a target trait from a segregating population obtained by the cross. Further, with the present invention, data of chromosomal recombinations in the hybrid generations can be accumulated, making it possible to readily estimate recombinations. This enables gene groups to be inserted in units of blocks, or genes in the blocks to be modified.

The organisms to which the identification or screening of a genotype according to the present invention is applicable are not particularly limited, and may be any of plants, animals, and microorganisms. Particularly, the invention is applicable to organisms that have chromosomes and follow the laws of Mendelian genetics. The organisms that follow the laws of Mendelian genetics are not particularly limited, and those commercially available and for which need for variety improvement is high can be used.

In the case of plants, various crops (plant and farm products produced in agriculture, forestry, and fishery industries) can be used. Specific examples include: cereals such as rice, wheat, barley, rye, triticale, and corn; marine plants such as seaweed; various vegetables and flowers; and trees such as cedar or cypress. In the case of animals, various domestic animals can be used. Specific examples include: domestic mammals such as bovines, sheep, and pigs; domestic birds such as chickens and quails; fish such as yellowtail snapper, sea bream, carp, and sweetfish; insects such as honey bees, and silkworm; and shellfish such as oyster, ormer, and scallop. As microorganisms, bacteria such as *Escherichia coli*, yeasts, fungi, actinomycetes, and basidiomycetes can be used.

Among these examples, the cereals include crops such as rice, wheat, corn, and barley, which are cultivated worldwide and are strategically important. Thus, by using the present invention for the variety improvement of these plants, varieties with desirable traits can be efficiently produced.

An array according to the present invention can also be used for experimental animals and plants. Specific examples of experimental animals include mice, rats, D. *melanogaster,* and C. *elegans.* A specific example is *Arabidopsis thaliana.*

Further, for the purpose of identifying genotypes by the present invention, the invention can be applied to humans.

It should be appreciated that the present invention is not just limited to the foregoing embodiments. The foregoing examples are not intended to limit the invention to the particular forms disclosed, but on the contrary, the invention is to cover all modifications, equivalents, and alternatives falling within the scope of the invention as defined in the appended claims.

As described, in a genotype analyzing and display system according to the present invention, comprehensive gene-expression-level information of hybrid individuals is compared with the genetic information of parents of the hybrid individuals, and a genetic map of the species to which these individuals belong, so as to determine and display the origin of the genotype of a hybrid individual of interest. Thus, for each chromosome of the hybrid individuals, locations of crossovers can be graphically displayed. That is, a genotype of a hybrid individual can be accurately determined or recognized only by acquiring nucleic acid from each individual of the hybrid generation and obtaining a hybridization result using the nucleic acid array.

Thus, with the present invention, whether or not a genotype or trait of interest conferred by such a genotype has been inherited can be accurately determined for each individual of the hybrid generation. This enables individuals with a target trait to be selected from a large group of hybrid generation both easily and reliably and with good repeatability. That is, the invention allows gene expression data obtained with the use of a nucleic acid array to be effectively used in crossing for variety improvement.

### [Fifth Embodiment]

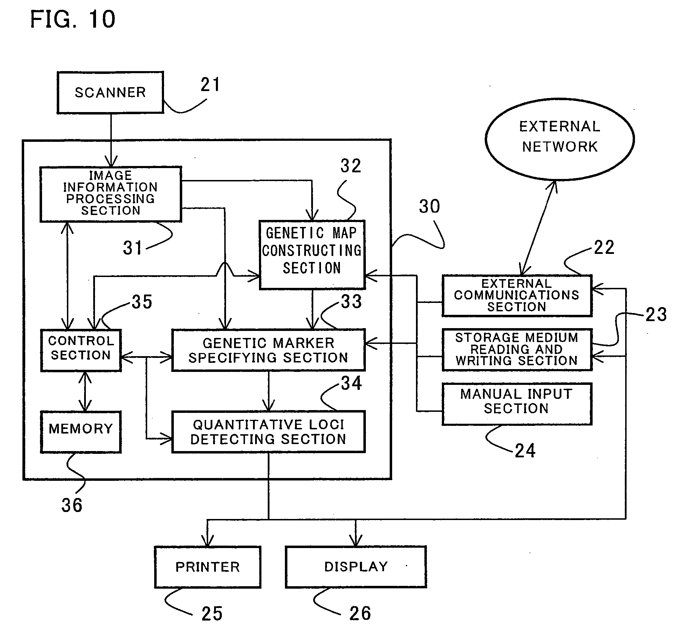

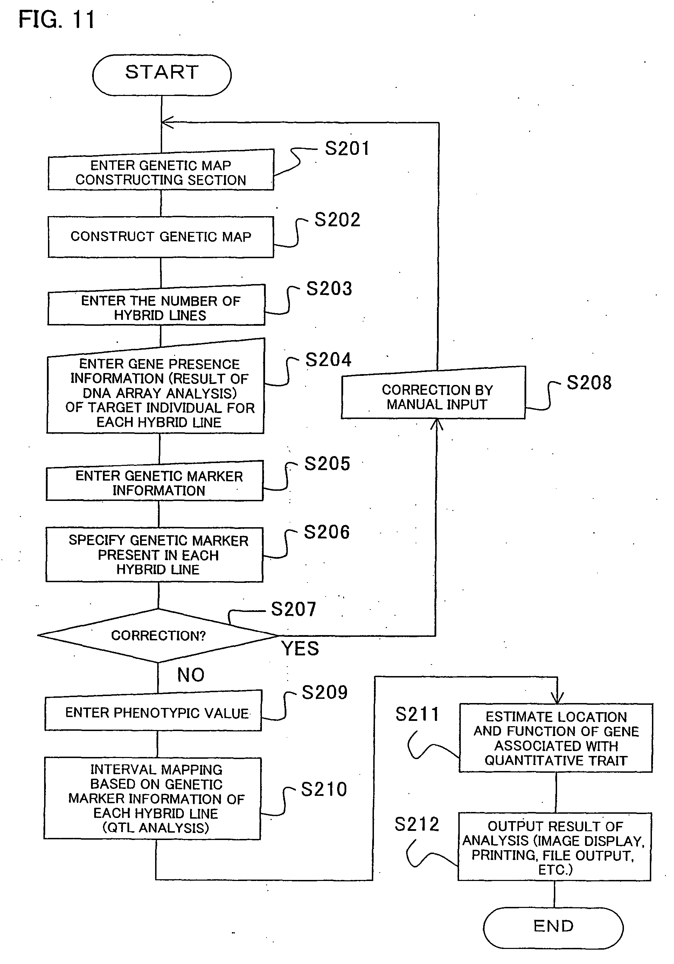

Referring to Fig. 10 and Fig. 11, the following will describe one embodiment of a quantitative loci analyzing system according to the present invention. It should be appreciated that the invention is not limited by the following description.

A quantitative loci analyzing system according to the present invention examines various Mendelian segregation populations, such as an F2 population, backcross population, and doubled haploid population, in regard to a phenotypic value (for example, disease resistance quantified in scores) of each hybrid individual of these populations, and hybridizes extracted nucleic acid samples of the hybrid individuals with a nucleic acid array so as to analyze quantitative loci using the array spots as genetic markers.

A quantitative loci analyzing system according to the present invention is not limited to a particular structure. For example, a quantitative loci analyzing system includes, as shown in Fig. 10, an image information processing section (image information processing means) 31, a genetic map constructing section (genetic map constructing means) 32, a genetic marker specifying section (genetic marker specifying means) 33, a quantitative loci detecting section (quantitative loci detecting means) 34, a control section (control means) 35, a memory (storage means) 36, a scanner (image reading means, input means) 21, an external communications section (external information input and output means) 22, a storage medium reading and writing section (storage means, input means, output means) 23, a manual input section (manual input means) 24, a printer (image forming means, printing means, output means) 25, and a display (image display means, output means) 26. The quantitative loci analyzing system having such a structure can be roughly divided into an input section, an output section, and an analyzing section (analyzing means) 30.

### (I) Nucleic acid array

The invention prepares genomic samples from hybrid individuals obtained from each different Mendelian segregation population of a desired species of living organism, and hybridizes the genomic samples with a nucleic acid array so as to obtain comprehensive gene-presence-information of the hybrid individuals. The nucleic acid array used in the present invention is not particularly limited, and conventional nucleic acid arrays can be suitably used. Specific examples include a micro array, a macro array, and a bead array. In the present embodiment, DNA is used as the nucleic acid, and therefore more specific examples of the nucleic acid array are DNA arrays such as DNA micro array and DNA macro array.

As to the specifics of the DNA micro array, DNA macro array, and other DNA arrays, no further description will be given since they were described in detail in the foregoing Fourth Embodiment. The chromosomal location recognizable array is preferably used also in the present embodiment.

The following describes a method of using the DNA array, taking the DNA micro array as an example. First, the DNA micro array is hybridized with fluorescent-labeled target DNA (hereinafter, "targets"). Here, the target molecules containing complementary sequences to the probes on the DNA micro array bind to (hybridize with) their complementary probe molecules, leaving other target molecules unbound. Then, these target molecules not bound to the probes are washed and removed, leaving only the hybridized target molecules on the micro array. Since the target molecules are fluorescence-labeled, the fluorescence of the targets is measured as signal intensity, so as to identify hybridized probes.

In the present invention, comprehensive gene-presence-information of hybrid individuals is obtained, and therefore the presence of individual genes can be recognized by the presence or absence of hybridization. Specifically, genomic DNA obtained from individuals are treated with restriction enzymes for example, and the resulting fragments are used as targets and hybridized with a DNA array so as to check for the presence or absence of complementary base sequences that hybridize with the probes on the DNA array. The resulting information is obtained as comprehensive gene-presence-information.

The nucleic acid molecules immobilized on the nucleic acid array are not particularly limited and DNA is generally used, as described above. The type of DNA used as probes immobilized on the DNA array (nucleic acid array) is not particularly limited either, and a genetic marker or a group of genetic markers are used in the invention. The genetic marker or a group of genetic markers are arranged based on a genetic map or physical map.

The genetic marker or a group of genetic markers are not particularly limited as long as they can serve as genetic labels on the chromosome. Non-limiting examples include an EST marker, a SNP marker including, a RFLP marker, and a micro satellite marker (SSR marker), as described above. Among these examples, a SNP marker and RFLP marker including polymorphism can be preferably used.

### (II) Structure of quantitative loci analyzing system

### <Input section>

In the present invention, genomic samples obtained from hybrid individuals of each different hybrid line are hybridized with a nucleic acid array on which genetic markers of a species have been immobilized. With the resulting comprehensive gene-presence-information of the hybrid individuals, genetic markers are specified for each different hybrid line and QTL analysis is carried out. To this end, a quantitative loci analyzing system according to the present invention includes, as an input section, means for entering comprehensive gene-presence-information for specifying genetic markers.

In the structure shown in Fig. 10, the analysis result from the nucleic acid array is entered as image information through the scanner 21. The image information processing section 31 analyzes the image information and generates gene-presence-information from the analysis result. The scanner 21 is not particularly limited as long as it can serve as image reading means for reading the hybridization result as image information. Specifically, the fluorescence of the targets that hybridized with the probes is read out as image data from the nucleic acid array, and the expression level of genes is detected from the signal intensity of the image data. Thus, as the scanner 21, a conventional fluorescent scanner 21 can be suitably used, for example.

Here, the image information obtained from the scanner 21 is subjected to necessary information processing to generate gene-presence-information. Thus, as shown in Fig. 10, the present invention preferably includes the image information processing section 31 for analyzing the image information, and generating comprehensive gene-presence-information. The image information processing section 31 is not limited to a particular structure, and conventional analyzing systems can be used.

Further, in the present invention, the result of hybridization analysis from the nucleic acid array (comprehensive gene-presence-information of the hybrid individuals) is compared with the genetic marker information of the hybrid individuals, and with the genetic map of the species to which these individuals belong, as will be described later. To this end, a quantitative loci analyzing system according to the present invention includes, in addition to the scanner 21 (image reading means), means for inputting at least one of the genetic marker information, and a phenotypic value representing a phenotype of interest. In addition, the quantitative loci analyzing system includes means for inputting at least one of a genetic map and genetic map constructing information.

The means for inputting genetic marker information or phenotypic value is not particularly limited. In the structure shown in Fig. 10, the external communications section 22, the storage medium reading and writing section 23, and the manual input section 24 correspond to such means. These input means are also used for inputting a genetic map or genetic map constructing information.

The external communications section 22 is not particularly limited as long as it allows for input and output of information to and from external devices, and conventional communications interfaces such as a LAN card, a LAN board, a LAN adapter, and a modem can be used. The storage medium reading and writing section 23 is not limited to a particular structure either. For example, known disk drives such as a hard disk drive, a flexible disk drive, a CD-ROM drive, and a DVD-ROM drive, or various memory cards or memory cartridges such as USB memory can be used. The manual input section 24 is not limited to a particular structure, and conventional input means such as a keyboard or a tablet can be suitably used.

An example of the genetic marker information is position information immobilized on the nucleic acid array. Specifically, the hybridization detects spots if nucleic acid molecules having complementary base sequences are present. Thus, once the positions of immobilized spots on the nucleic acid array were found to correspond to which genetic markers, the information can be used as genetic marker information.

The phenotypic value is not particularly limited as long as it represents a phenotype of interest. For example, the inventors of the present invention have evaluated resistance to Fusarium head blight with the scores of 0 (resistance) to 10 (diseased) by modifying a cut spike test (see *Development of Fusarium head blight testing method, and a search for resistant varieties in barley,* Japanese Journal of Variety Improvement, 39, 1989, Kazuyoshi Takeda, Hideo Heta). In this manner, phenotypic values may be suitably selected depending on the type of species to be analyzed, or the type of desired trait.

A relatively large number of genetic maps as chromosome maps are available for experimental animals and some of the crops and domestic animals. However, the selection of genetic maps is often not sufficient in most crops and domestic animals. Thus, the genetic map is directly entered if it is available. If not, genetic map constructing information is entered and a new genetic map is constructed in the genetic map constructing section 32 shown in Fig. 10. Details of the genetic map constructing section 32 will be described later.

A quantitative loci analyzing system according to the present invention preferably includes means for correcting at least one of the comprehensive gene-presence-information of hybrid individuals, genetic marker information, and genetic map constructing information. Specifically, the manual input section 24 in the structure shown in Fig. 10 corresponds to such means.

As will be described later, a quantitative loci analyzing system according to the present invention performs the step of checking for the presence or absence of an entry error in the analysis process, particularly in specifying genetic markers. This improves reliability of final interval mapping performed in a subsequent stage. It is therefore preferable that the system include means for correcting entry error, i.e., the manual input section 24, for example. Note that, the means for correcting entry error is not just limited to the manual input section 24, and other means may be used as well.

### <Analyzing section>

In the present invention, genomic samples obtained from hybrid individuals of each different hybrid line are hybridized with a nucleic acid array on which genetic markers of a species of interest have been immobilized. With the resulting comprehensive gene-presence-information of the hybrid individuals, genetic markers are specified for each different hybrid line and QTL analysis is carried out. To this end, a quantitative loci analyzing system according to the present invention includes, as an input section, means for entering comprehensive gene-presence-information for specifying genetic markers. Thus, the analyzing section 30 for analyzing the information entered through the input section is an essential component. The analyzing section 30 at least includes the genetic marker specifying section 33 and the quantitative loci detecting section 34, as shown in Fig. 10.

In the genetic marker specifying section 33, the gene-presence-information generated in the image information processing section 31 through the scanner 21 is compared with the genetic map and genetic marker information, so as to specify genetic markers for each different hybrid line. Specifically, from the result of comparison between a genetic map and position information of the genetic markers immobilized on the nucleic acid array, whether or not a hybrid individual of interest includes the genetic markers is determined. If the genetic markers are included, the genetic markers are specified as the genetic markers of the hybrid line to which the hybrid individuals belong.

In obtaining the presence information of genes of the hybrid individuals, the use of the chromosomal location recognizable array as a nucleic array allows the order of immobilized spots to be used as the genetic marker information. In other words, the order of immobilized spots and the map distance (cM) of chromosomes, etc. can be used as genetic marker information. This is highly preferable as it makes it easier for the genetic marker specifying section 33 to perform the comparison. Further, as described above, the genetic marker information, should preferably be polymorphic genetic markers (SNP or RFLP), since it is easily recognizable as typical genetic markers of each different hybrid line.

The quantitative loci detecting section 34, determines the quantitative loci of a phenotype of interest in the same hybrid individuals by checking whether a phenotypic value representing the phenotype is linked to the specific genetic markers. The quantitative loci are determined by interval mapping. The interval mapping is not particularly limited, and simple interval mapping (SIM), or composite interval mapping (CIM) may be used, for example. For a specific analysis of interval mapping, a known analyzing system may be used. Specific examples of such analyzing system include those using analyzing software such as MAPMARKER/QTL or QTL Cartographer.

A quantitative loci analyzing system according to the present invention may include the genetic map constructing section 32 in addition to the genetic marker specifying section 33 and the quantitative loci detecting section 34. Based on the genetic map constructing information, the genetic map constructing section 32 constructs a genetic map of a species to which the hybrid individuals belong. As described earlier, the genetic map is constructed for only some of the species. It is therefore preferable to provide the genetic map constructing section 32.

The genetic map constructing section 32 is not particularly limited as long as the genetic map is constructed on the chromosome basis based on various genetic map constructing information. As the genetic map constructing information, at least names of genes and/or genetic markers known in the species being analyzed, and chromosomal loci of the genes and/or genetic markers are used, for example.

The means for entering the genetic map constructing information is not particularly limited, and various input sections, for example, such as the external communications section 22, the storage medium reading and writing section 23, and the manual input section 24 shown in Fig. 10 can be used.

Further, with the chromosomal location recognizable array, a genetic map can be constructed through mapping of genetic markers with unknown locations. Specifically, in order to construct a genetic map, targets obtained from a Mendelian segregation population of the species being analyzed are hybridized with the chromosomal location recognizable array. Then, genetic markers with unknown locations are hybridized on the same chromosomal location recognizable array, so as to determine locations of the genetic markers. In this way, a highly dense genetic map can be constructed.

Even though the foregoing example uses the same chromosomal location recognizable array, the method of mapping the genetic markers of unknown locations is not just limited to this example. For example, mapping can be made by processing the same targets with the genetic markers on different arrays. Here, mapping of genes is possible if the genes follow the rule of Mendelian segregation as in Single Feature Polymorphism (SFP), even if SNP or RFLP is not detected.

Thus, a quantitative loci analyzing system according to the present invention may be adapted so that, in order to construct a genetic map in the genetic map constructing section 32, the hybridization result is analyzed and processed by reading it from the array with the scanner 21 and the image information processing section 31, before analyzing genotypes. To this end, the image information processing section 31 is adapted to output information also to the genetic map constructing section 32, as shown in Fig. 10 (as indicated by arrow in the figure).

Information such as the genetic map constructed by the genetic map constructing section, 32, information concerning the genetic markers identified by the genetic marker specifying section 33, and the result of determination made by the genotype origin detecting section 34 can be temporarily stored in the memory 36. The memory 36 is provided in the analyzing section 30 as shown in Fig. 10, and serves as a storage section for storing various information used or generated in a quantitative loci analyzing system according to the present invention. The storage operation of the memory 36 is controlled by the control section 35. The memory 36 is not limited to a particular structure, and may be realized, for example, by a semiconductor memory, such as RAM or ROM. Note that, the storage medium reading and writing section 23 described as an input section can be used as a storage section of the present invention. This will be described later in more detail in conjunction with the output section.

The analyzing section 30 of the structure shown in Fig. 10 includes the control section 35 for controlling the entire operation of the analyzing section 30, and in turn the entire operation of the quantitative loci analyzing system. In the structure shown in Fig. 10, the control section 35 outputs control information to the image information processing section 31, the genetic map constructing section 32, the genetic marker specifying section 33, the display information generating section 34, and the memory 36. These means operate based on the control information they receive, thereby operating the quantitative loci analyzing system. It should be noted here that the control section 35 is also adapted to receive information from these means, and as such the flow of control information is indicated by the bidirectional arrow in Fig. 10.

### <Output section>

In the present invention, a genomic sample of a hybrid individual obtained from each different hybrid line is hybridized with a nucleic acid array on which genetic markers of a species of interest are immobilized. In this way, comprehensive gene-presence-information of hybrid individuals are obtained, and by using the gene-presence-information, genetic markers are specified for each different hybrid line and QTL analysis is performed. To this end, a quantitative loci analyzing system according to the present invention includes means, provided as an output section, for outputting a result of QTL analysis.

The output section is not particularly limited, and at least one of, or preferably both of a display 26 for displaying a result of QTL analysis on a display screen (soft copy), and a printer 25 for printing a result of QTL analysis (hard copy) are provided. The display 26 is not limited to a particular structure, and various types of known displays such as a CRT, a liquid crystal display, and a plasma display can be used. The printer 25 is not limited to a particular structure, and known image forming devices such as an ink-jet printer and laser printer can be used.

The output section is not just limited to the display 26 or printer 25, and other means can be used as well. For example, the external communications section 22 can be used as an output section. Specifically, the external communications section 22 allows for input and output of information to and from external devices by serving as both an input section and an output section. This enables the result of QTL analysis to be transmitted to other devices via external networks, etc, enabling a quantitative loci analyzing system according to the present invention to be used more efficiently.

Specifically, when the quantitative loci analyzing system is connected to external devices via LAN for example, the quantitative loci analyzing system, installed in a research facility for example, can be shared with other researchers via information terminals such as personal computers. Further, the results of analysis obtained in the quantitative loci analyzing system may be accumulated in an external server via a communications network, allowing the analysis result to be used more efficiently.

As the output section, the storage medium reading and writing section 23, described as an input section, can be suitably used. Specifically, in a quantitative loci analyzing system according to the present invention, a drive for reading information from a storage medium can be used as an output section if the drive has a writing capability. The storage medium reading and writing section 23 is not limited to a particular structure, and known disk drives such as a hard disk drive, a flexible disk drive, a CD-ROM drive, and a DVD-ROM drive, or various memory cards or memory cartridges such as a USB memory can be suitably used for example, as described above in conjunction with the input section.

Note that, in the exemplary structure shown in Fig. 10, the system is realized by the analyzing section 30 and independently provided input and output sections, wherein the analyzing section 30 includes the image information processing section 31, the genetic map constructing section 32, the genetic marker detecting section 33, the quantitative loci detecting section 34, the control section 35, and the memory 36. However, the present invention is not just limited to this structure. For example, all means may be provided as a single unit, or some of the input sections and/or output sections may be integrated with the analyzing section 30. Further, the system may include means other than those shown in Fig. 10.

The analyzing section 30 is not just limited to a particular structure, and conventional arithmetic means, for example, such as a central processing unit (CPU) of a computer may be used. The operation of the analyzing section 30 is executed by a computer program.

### (III) Analyzing method by the quantitative loci analyzing system

An analyzing method performed by a quantitative loci analyzing system according to the present invention is not particularly limited. Specifically, the method may include 12 steps as represented in Fig. 11.

First, in step 201 (step will be denoted by "S" hereinafter), genetic map constructing information (names of chromosomes, genes, and genetic markers, and loci, etc.) is entered through input sections. In S202, the genetic map constructing section 32 constructs a genetic map based on the genetic map constructing information, and the genetic map is supplied to the genetic marker detecting section 33. In S203, the number of hybrid lines is entered through the input section. In S204, gene-presence-information (i.e., the result of DNA array analysis) of the hybrid individuals (targets individuals in Fig. 11) being analyzed is entered through the scanner 21 and the image information processing section 31 for each different hybrid line. In S205, genetic marker information is entered.

In S206, based on the entered information, the genetic marker detecting section 33 determines a genetic marker that is present in each different hybrid line. Here, the result of determination of a genetic marker may be stored in the memory 36, or optionally displayed in the display 26. In S207, the presence or absence of an entry error is found (need for correction is determined). If there is an entry error (YES), the information is re-entered in S208 through, for example, the manual input section 24, and the sequence returns to S201. On the other hand, if correction is not required (NO), the sequence goes to S209.

In S209, a phenotypic value is entered through the input section. In S210, interval mapping (QTL analysis) is performed based on the result of determination of a genetic marker and the phenotypic value, so as to determine a quantitative locus of the phenotype. In S211, based on the result of QTL analysis, the locations and functions of the genes associated with the quantitative traits are estimated. The result of analysis is outputted from the output section in S212. This completes the series of analysis procedures.

### (IV) Use of the present invention

Use of a quantitative loci analyzing system according to the present invention is not particularly limited as long as QTL analysis is carried out on a species of interest. In one specific example, a search for a useful gene is made through an organism of interest, and such a useful gene is used for variety improvement. That is, in the present invention, the quantitative loci analyzing system can be suitably used in a quantitative trait analyzing method in which quantitative traits of organisms are analyzed, and in a gene search method in which genes associated with expression of traits of interest are searched.

A QTL analysis is an analyzing method by which a genetic map distance between two loci is estimated based on recombination value. The genetic map distance refers to an expected value of a crossover between two loci that occurs in each round of meiosis. In the QTL analysis, a recombination value is estimated from the data of hybrid lines, and a genetic map distance is estimated from the recombination value.

If it is assumed that crossing over occurs at equal probability anywhere on the chromosomes, then the genetic map distance is directly proportional to the physical distance. From this relationship, the physical distance can be estimated based on the genetic map distance. By thus finding the genetic map distance between a genetic marker of a known physical location and a gene associated with the expression of the trait of interest, the chromosomal location of the gene can be specified with fair accuracy.

It should be noted, however, that the recombination value is equal to the genetic map distance only when the physical distance between two loci on the chromosome is close. That is, the recombination value does not indicate the genetic map distance when the two loci are far apart from each other. The number of crossovers that might have occurred between two loci cannot be directly measured, and it is measurable only through recombination values. Thus, a genetic map distance is estimated from the recombination value, using a genetic map function.

A QTL analysis is generally performed in the following manner. First, genetically different two hybrid lines are crossed to produce hybrid generations F1, F2 and subsequent generations. Then, typing of multiple genetic markers is performed for these individuals, and statistics are taken for the resulting data.

As described thus far, the QTL analysis requires estimation of a genetic map distance, or processing of typing data of genetic markers, etc. The QTL analysis is therefore suitable for technical areas involving bioinformatics. To this date, no proposal has been made for applying QTL analysis to these technical areas. As described above, in the present invention, a genomic sample of hybrid individuals obtained from each different hybrid, line is hybridized with a nucleic acid array on which genetic markers of the species of interest are immobilized, using a quantitative loci analyzing system. With the resulting comprehensive gene-presence-information of the hybrid individuals, genetic markers are specified for each different hybrid line and QTL analysis is carried out. In this way, information can be analyzed comprehensively in the QTL analysis, making it possible to efficiently perform the QTL analysis.

Thus, a more specific application of the present invention is use of the quantitative loci analyzing system in variety improvement using genetic markers.

The type of organism to which a quantitative trait analyzing method, gene search method, or variety improvement method of the present invention is applicable is not particularly limited, and any of plants, animals, and microorganisms may be used. Particularly, the present invention is applicable in organisms that include chromosomes and obey the laws of Mendelian genetics. Examples of such an organism are, but not limited to, those commercially available and for which need for variety improvement is high.

In the case of plants, various crops (plant and farm products produced in agriculture, forestry, and fishery industries) can be used. Specific examples include: cereals such as rice, wheat, barley, rye, triticale, and corn; marine plants such as seaweed; various vegetables and flowers; and trees such as cedar or cypress. In the case of animals, various domestic animals can be used. Specific examples include: domestic mammals such as bovines, sheep, and pigs; domestic birds such as chickens and quails; fish such as yellowtail snapper, sea bream, carp, and sweetfish; insects such as honey bees, and silkworm; and shellfish such as oyster, ormer, and scallop. As microorganisms, bacteria such as *Escherichia coli*, yeasts, fungi, actinomycetes, and basidiomycetes can be used.

Among these examples, the cereals include crops such as rice, wheat, corn, and barley, which are cultivated worldwide and are strategically important. Thus, by using the present invention for the variety improvement of these plants, varieties with desirable traits can be efficiently produced. The invention is also applicable to experimental animals and plants. Specific examples of experimental animals include mice, rats, *D*. *melanogaster,* and *C*. *elegans.* A specific example is *Arabidopsis thaliana.* Further, for the purpose of identifying genotypes with the present invention, the invention can be applied to humans.

It should be appreciated that the present invention is not just limited to the foregoing embodiments. The foregoing examples are not intended to limit the invention to the particular forms disclosed, but on the contrary, the invention is to cover all modifications, equivalents, and alternatives falling within the scope of the invention as defined in the appended claims.

As described above, a quantitative loci analyzing system according to the present invention utilizes a hybridization analysis using a nucleic acid array for specifying genetic markers present in each hybrid line, and performs QTL analysis using the genetic markers so specified. In this way, information can be analyzed comprehensively in the QTL analysis, making it possible to efficiently perform QTL analysis.

### [Sixth Embodiment]

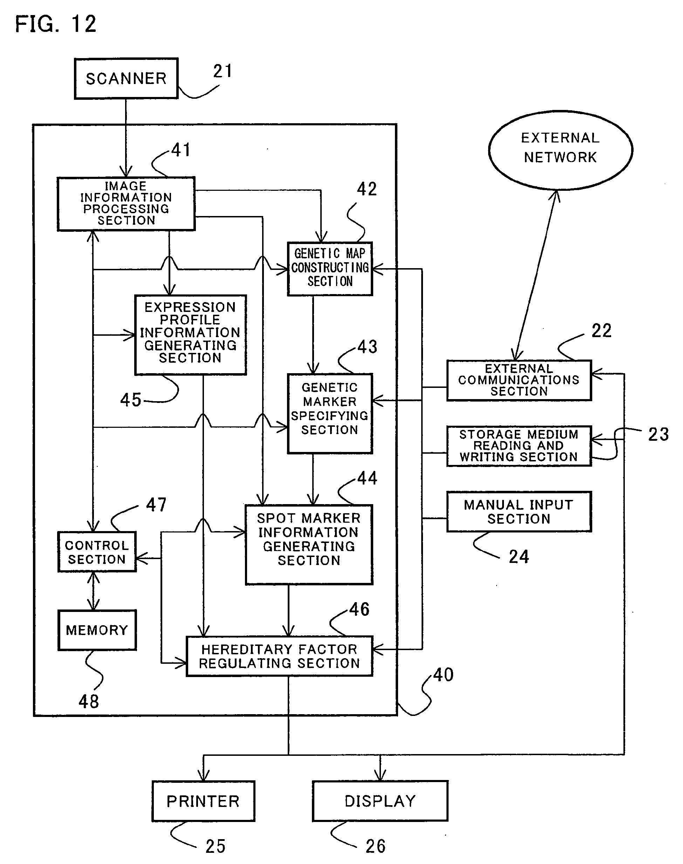

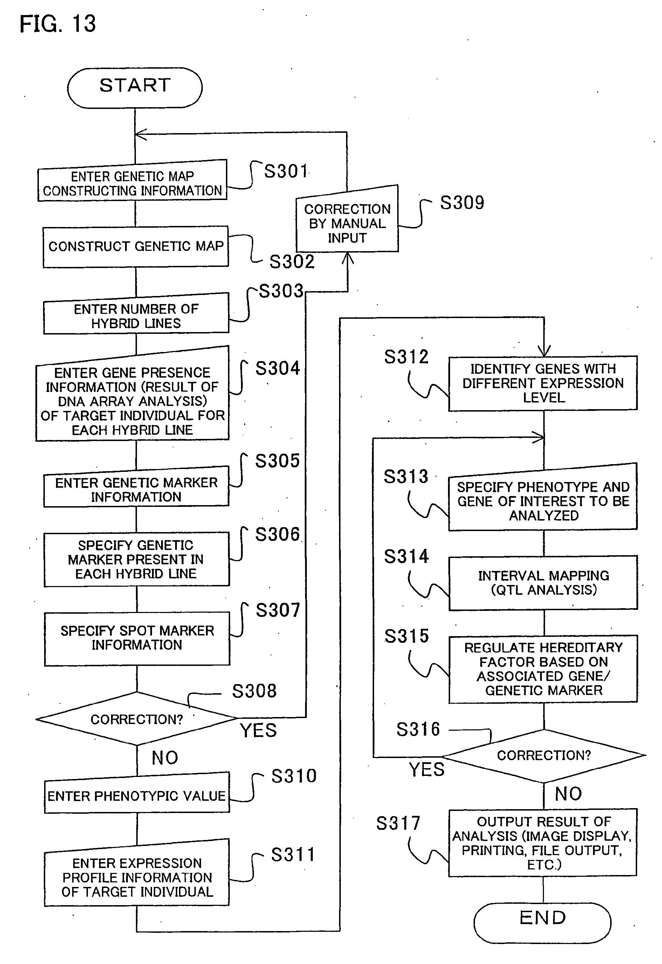

Referring to Fig. 12 and Fig. 13, the following will describe another embodiment of the present invention. It should be appreciated that the invention is not limited by the following description.

A gene interaction analyzing system according to the present invention individually performs interval mapping (QTL analysis) for specific genes or phenotypes based on the hybridization result of genetic markers immobilized on a nucleic acid array, and thereby estimates locations and functions of genes involved. In this way, hereditary factors of specific genes or phenotypes can be regulated, allowing for efficient analysis of gene interaction.

A gene interaction analyzing system according to the present invention is not limited to a particular structure. For example, a gene interaction analyzing system includes, as shown in Fig. 12, an image information processing section (image information processing means) 41, a genetic map constructing section (genetic map constructing means) 42, a genetic marker specifying section (genetic marker specifying means) 43, a spot marker information generating section (spot marker information generating means) 44, an expression profile information generating section (expression profile information generating means) 45, a hereditary factor regulating section (hereditary factor regulating means) 46, a control section (control means) 47, a memory (storage means) 48, a scanner (image reading means, input means) 21, an external communications section (external information input and output means) 22, a storage medium reading and writing section (storage means, input means, output means) 23, a manual input section (manual input means) 24, a printer (image forming means, printing means, output means) 25, and a display (image display means, output means) 26. The gene interaction analyzing system having such a structure can be roughly divided into an input section, an output section, and an analyzing section (analyzing means) 40.

### (I) Nucleic acid array

The invention prepares genomic samples from hybrid individuals obtained from each different Mendelian segregation population of a desired species of living organism, and hybridizes the genomic samples with a nucleic acid array so as to obtain comprehensive gene-presence-information of the hybrid individuals. The comprehensive gene-presence-information so obtained is used for the analysis of gene interaction. The nucleic acid array used in the present invention is not particularly limited, and conventional nucleic acid arrays can be suitably used. Specific examples include a micro array, a macro array, and a bead array. In the present embodiment, DNA is used as the nucleic acid, and therefore more specific examples of the nucleic acid array are DNA arrays such as DNA micro array and DNA macro array.

As to the specifics of the DNA arrays, no further description will be given since they are described in detail in the foregoing Fourth and Fifth Embodiments. The chromosomal location recognizable array is preferably used also in the present embodiment.

The following describes a method of using the DNA array, taking the DNA micro array as an example. First, the DNA micro array is hybridized with fluorescent-labeled target DNA (hereinafter, "targets"). Here, the target molecules containing complementary sequences to the probes on the DNA micro array bind to (hybridize with) their complementary probe molecules, leaving other target molecules unbound. Then, these target molecules not bound to the probes are washed and removed, leaving only the hybridized target molecules on the micro array. Since the target molecules are fluorescence-labeled, the fluorescence of the targets is measured as signal intensity, so as to identify hybridized probes.

In the present invention, comprehensive gene-presence-information of hybrid individuals is obtained, and therefore the presence of individual alleles can be recognized by the presence or absence of hybridization. Specifically, genomic DNA obtained from individuals are treated with restriction enzymes for example, and the resulting fragments are used as targets and hybridized with a DNA array so as to check for the presence or absence of complementary base sequences that hybridize with the probes on the DNA array. The resulting information is obtained as comprehensive gene-presence-information.

### (II) Structure of gene interaction analyzing system

### <Input section>

In the present invention, comprehensive gene-presence-information of hybrid individuals is compared with a genetic map of the species to which the hybrid individuals belong, and with the genetic marker information known in the species, so as to specify genetic markers present in each hybrid line and thereby generate spot marker information (described later) based on the genetic markers so specified. Then, phenotypes and genes of interest to be analyzed are specified, and whether or not phenotypic values representing the phenotypes are linked to the expressed genes included in the expression profile information obtained from the same hybrid individuals is confirmed. To this end, a gene interaction analyzing system according to the present invention includes, as an input section, means for entering at least one of comprehensive gene-presence-information of hybrid individuals, the genetic marker information, the phenotypic values, and the expression profile information.

The means for entering the comprehensive gene-presence-information is not particularly limited. For example, in the structure shown in Fig. 12, the analysis result from the nucleic acid array is entered as image information through the scanner 21. The image information processing section 41 analyzes the image information and generates gene-presence-information from the analysis result. In this manner, the scanner 21 can be used as the means for entering comprehensive gene-presence-information, for example.

The scanner 21 is not particularly limited as long as it can serve as image reading means for reading the nucleic acid array hybridization result as image information. Specifically, the fluorescence of the targets that hybridized with the probes is read out as image data from the nucleic acid array, and the expression level of genes is detected from the signal intensity of the image data. Thus, as the scanner 21, a conventional fluorescent scanner 21 can be suitably used, for example.

Here, the image information obtained from the scanner 21 is subjected to necessary information processing to generate gene-presence-information. Thus, as shown in Fig. 12, the present invention preferably includes the image information processing section 41 for analyzing the image information, and generating comprehensive gene-presence-information. The image information processing section 41 is not limited to a particular structure, and conventional analyzing systems can be used.

The means for entering the genetic marker information and phenotypic value is not particularly limited. In the structure shown in Fig. 12, for example, the external communications section 22, the storage medium reading and writing section 23, and the manual input section 24 correspond to such means. The expression profile information can be generated by reading the result of nucleic acid array analysis, as will be described later. Alternatively, expression profile information that has been obtained by performing expression profile analysis beforehand may be entered as appropriate. Thus, the external communications section 22 and the storage medium reading and writing section 23, etc. can also be used as the means for entering the expression profile information.

Further, the present invention uses a genetic map for specifying genetic markers that exist in each hybrid line. To this end, it is preferable that means be provided for entering the genetic map or genetic map constructing information, as well as the foregoing information. As the means for entering the genetic map or genetic map information, the external communications section 22, the storage medium reading and writing section 23, and the manual input section 24 can be suitably used, for example.

The external communications section 22 is not particularly limited as long as it allows for input and output of information to and from external devices, and conventional communications interfaces such as a LAN card, a LAN board, a LAN adapter, and a modem can be used. The storage medium reading and writing section 23 is not limited to a particular structure either. For example, known disk drives such as a hard disk drive, a flexible disk drive, a CD-ROM drive, and a DVD-ROM drive, or various memory cards or memory cartridges such as USB memory can be used. The manual input section 24 is not limited to a particular structure, and conventional input means such as a keyboard or a tablet can be suitably used.

An example of the genetic marker information is position information immobilized on the nucleic acid array. Specifically, hybridization detects spots if nucleic acid molecules having complementary base sequences are present. Thus, once the positions of immobilized spots on the nucleic acid array were found to correspond to which genetic markers, the information can be used as genetic marker information.

The phenotypic value is not particularly limited as long as it represents a phenotype of interest. For example, the inventors of the present invention have evaluated resistance to Fusarium head blight with the scores of 0 (resistance) to 10 (diseased) by modifying a cut spike test (see *Development of Fusarium head blight testing method, and a search for resistant varieties in barley,* Japanese Journal of Variety Improvement, 39, 1989, Kazuyoshi Takeda, Hideo Heta). In this manner, phenotypic values may be suitably selected depending on the type of species to be analyzed, or the type of desired trait.

A relatively large number of genetic maps as chromosome maps are available for experimental animals and some of the crops and domestic animals. However, the selection of genetic maps is often not sufficient for most crops and domestic animals. Thus, the genetic map is directly entered if it is available. If not, genetic map constructing information is entered and a new genetic map is constructed in the genetic map constructing section 42 shown in Fig. 12. Details of the genetic map constructing section 42 will be described later.

The expression profile information is not particularly limited as long as it is obtained by comprehensively analyzing gene expression in the cell. As noted earlier, information that has been analyzed may be entered as the expression profile information; however, it is more preferable that expression profile analysis be performed as appropriate for the hybrid individuals being analyzed. Thus, the means for entering expression profile information can be used not only to enter analyzed information but to generate expression profile information by reading and analyzing the expression profile as appropriate.

As the means for reading the expression profile, image reading means such as the scanner 21 can be used. The experiment system used for reading (experiment system for performing an expression profile experiment) is not particularly limited, and a nucleic acid array for obtaining comprehensive gene-presence-information may be used. Other than the nucleic acid array, other experiment systems can be used as well. Examples of the nucleic acid array include a micro array, a macro array, and a bead array, as described earlier. As an experiment system for performing an expression profile experiment, a differential display may be used, for example.

A differential display is a technique whereby a difference in gene expression level in the cells placed under different conditions is detected on a gel as a difference between band profiles, and the genes are collected and identified. Specifically, in the case of a fluorescent differential display for example, a PCR product of fluorescent-labeled cDNA is obtained from total RNA, and a fluorescent image is measured as signal intensity after separating the PCR product on a denatured polyacrylamide gel.

The differential display is not a method for comprehensively analyzing total mRNA. However, since the differential display allows a large number of samples to be compared using a small amount of mRNA, it can be used to perform the expression profile experiment as with the nucleic acid array. Thus, as the input section, image reading means for detecting signal intensity from a gel plate of the electrophorased polyacrylamide gel is provided.

A gene interaction analyzing system according to the present invention preferably includes means for correcting at least one of the comprehensive gene-presence-information of hybrid individuals, genetic marker information, and genetic map constructing information. Specifically, the manual input section 24 in the structure shown in Fig. 12 corresponds to such means.

As will be described later, a gene interaction analyzing system according to the present invention performs the step of checking for the presence or absence of an entry error in the analysis process, particularly in specifying genetic markers. This improves reliability of final interval mapping performed in a subsequent stage. It is therefore preferable that the system include means for correcting entry error, i.e., the manual input section 24, for example. Note that, the means for correcting entry error is not just limited to the manual input section 24, and other means may be used as well.

### <Analyzing section>

In the present invention, genetic markers are specified and spot marker information (described later) is generated from the genetic markers. Then, it is confirmed whether expressed genes in the expression profile information are linked to the spot marker information, so as to regulate hereditary factors of the phenotype of interest. To this end, a gene interaction analyzing system according to the present invention includes, as an essential component, the analyzing section 40 for analyzing the information entered through the input section. The analyzing section 40 includes at least the genetic marker specifying section 43, the spot marker information generating section 44, and the hereditary factor regulating section 46, as shown in Fig. 12.

In the genetic marker specifying section 43, the gene-presence-information generated in the image information processing section 41 through the scanner 21 is compared with the genetic map and genetic marker information, so as to specify genetic markers for each different hybrid line. Specifically, from the result of comparison between a genetic map and position information of the genetic markers immobilized on the nucleic acid array, whether or not a hybrid individual of interest includes the genetic markers is determined. If the genetic markers are included, the genetic markers are specified as the genetic markers of the hybrid line to which the hybrid individuals belong.

In obtaining the presence information of genes of the hybrid individuals, the use of the chromosomal location recognizable array as a nucleic array allows the order of immobilized spots to be used as the genetic marker information. In other words, the order of immobilized spots and the map distance of chromosomes, etc. can be used as genetic marker information. This is highly preferable as it makes it easier for the genetic marker specifying section 43 to perform the comparison. Further, as described above, the genetic marker information should preferably be polymorphic genetic markers (SNP or RFLP), since it is easily recognizable as typical genetic markers of each different hybrid line.

In the spot marker information generating section 44, the genetic markers specified by the genetic marker specifying section 43 are compared with the genetic markers immobilized on the nucleic acid array, and the result of hybridization of individual spots on the nucleic acid array is generated as spot marker information and used as genetic marker information for analysis. More specifically, the specified genetic markers are compared with the genetic markers on the nucleic acid array, and only the genetic marker spots so found by the hybridization are generated as spot marker information. This enables the hybridized spots on the nucleic acid array to be used as the genetic markers that exist in each hybrid line.

The chromosomal location recognizable array may be used as the nucleic acid array. In this case, the order of immobilized spots can be used as the genetic marker information. Specifically, in generating the spot marker information, certain immobilized genetic markers are obtained by reversing the order of immobilized spots, and the genetic markers so obtained are compared with the genetic markers previously specified. It is therefore preferable that the spot marker information include position information indicative of the positions of genetic markers immobilized on the nucleic acid array. In this way, when the expression profile information was obtained using the chromosomal location recognizable array, the position information can actually be used as spot marker information, and genetic markers can be readily specified.

In the hereditary factor regulating section 46, phenotypes and genes of interest to be analyzed are specified, and whether or not phenotypic values representing the phenotypes, and the expressed genes of interest included in the expression profile information obtained from the same hybrid individual are linked to plural pieces of spot marker information is confirmed. In this manner, the hereditary factors of the phenotypes of interest are regulated based on the expressed genes. Thus, based on the specified phenotypes and genes associated with the expression of the phenotypes, genes that are closely associated with the expression of the phenotype can be selected from the spot marker information, based on the presence or absence of a linkage.

Specifically, for a gene p1 known to be associated with a phenotype (trait) P, the gene p1 and a phenotypic value Vp representing the phenotype P are specified, and the presence or absence of any linkage between the spot marker information and phenotypic value Vp, and gene p 1 is confirmed. For example, genetic markers mp2 and mp3 are found as genetic marker information linked to the phenotypic value Vp and gene p1. Here, if these genetic markers mp2 and mp3 are for genes p2 and p3, then the genes p2 and p3 are specified as genes interacting with the phenotype P and gene p1. Note that, the genetic markers can determine hereditary factors even when there is no linkage to known genes. Thus, as the information for regulating hereditary factors of the phenotypes, the hereditary factor regulating section 46 uses at least genetic markers, and preferably uses known genes.

When no spot marker is found to be directly linked, a quantitative trait locus (QTL) is estimated between the most proximate genetic markers, and the hereditary factors may be regulated based on the QTL. Thus, provided that genetic markers to be used as spot marker information are present with such a density that linkage is detectable on the genetic map, hereditary factors can be regulated based on QTL even when genetic markers that are linked at high resolution cannot be specified.

By thus finding genetic markers (or genes, QTL) linked to the phenotypes, the hereditary factor regulating section 46, based on such genetic markers, can estimate locations or functions of genes associated with the expression of the phenotypes. Further, as the information for regulating hereditary factors of the phenotypes, the hereditary factor regulating section 46 can use the expression level of genes associated with the genetic markers.

Whether the plural pieces of spot marker information is linked to the specified phenotypic values or genes can be found through analysis employing interval mapping. The interval mapping is not particularly limited, and simple interval mapping (SIM), or composite interval mapping (CIM) may be used, for example. For a specific analysis of interval mapping, a known analyzing system may be used. Specific examples of such analyzing system include those using analyzing software such as MAPMARKER/QTL or QTL Cartographer.

A gene interaction analyzing system according to the present invention may include the expression profile information generating section 45 in addition to the genetic marker specifying section 43, the spot marker information generating section 44, and the hereditary factor regulating section 46. As described earlier, the expression profile information generating section 45 is adapted to generate expression profile information of a hybrid individual by performing expression profile analysis with regard to a comprehensive gene-expression level obtained from the same hybrid individual. Here, the expression profile information is generated through comprehensive measurement of gene expression using at least one of experiment systems using a micro array, a macro array, a bead array, and a differential display, as described above.

The expression profile information generating section 45 can also generate expression profile information using the nucleic acid array used for obtaining comprehensive gene-presence-information of hybrid individuals, or the nucleic acid array on which the same sample has been spotted. Namely, in the structure shown in Fig. 12, the scanner 21 first reads the result of expression profile experiment, and then the expression profile information generating section 45 generates expression profile information after images have been processed in the image processing section 11.

Here, the analysis can be carried out more efficiently when analysis data for specifying genetic markers (and for constructing a genetic map) and analysis data for the expression profile are acquired simultaneously. For example, when four or more kinds of labeling is possible in the hybridization experiment using the nucleic acid array, two kind of labeling may be set for specifying genetic markers, and two kinds for gene expression. In this way, analysis data for specifying genetic markers (and for constructing a genetic map) and analysis data for the expression profile can be acquired simultaneously from a single nucleic acid array.

Note that, in the structure shown in Fig. 12, the expression profile information generating section 45 and the image information processing section 41 may be provided in one unit. That is, the expression profile information may be generated by the image information processing section 41.

A gene interaction analyzing system according to the present invention may include the genetic map constructing section 42 in addition to the genetic marker specifying section 43, the spot marker information generating section 44, the expression profile information generating section 45, and the hereditary factor regulating section 46. Based on genetic map constructing information, the genetic map constructing section 42 constructs a genetic map of a species to which the hybrid individual belongs. As described earlier, the genetic map is constructed for only some of the species. It is therefore preferable to provide the genetic map constructing section 42.

The genetic map constructing section 42 is not particularly limited as long as the genetic map is constructed on the chromosome basis based on various genetic map constructing information. As the genetic map constructing information, at least names of genes and/or genetic markers known in the species being analyzed, and chromosomal loci of the genes and/or genetic markers are used, for example.

The means for entering the genetic map constructing information is not particularly limited, and various input sections, for example, such as the external communications section 22, the storage medium reading and writing section 23, and the manual input section 24 shown in Fig. 12 can be used.

Further, with the chromosomal location recognizable array, a genetic map can be constructed through mapping of genetic markers with unknown locations. Specifically, in order to construct a genetic map, targets obtained from a Mendelian segregation population of the species being analyzed are hybridized with the chromosomal location recognizable array. Then, genetic markers with unknown locations are hybridized on the same chromosomal location recognizable array, so as to determine locations of the genetic markers. In this way, a high density genetic map can be constructed.

Even though the foregoing example uses the same chromosomal location recognizable array, the method of mapping the genetic markers of unknown location is not just limited to this example. For example, mapping can be made by processing the same targets with the genetic markers on different arrays. Here, mapping of genes is possible if the genes follow the rule of Mendelian segregation as in Single Feature Polymorphism (SFP), even if SNP or RFLP is not detected.

Thus, a gene interaction analyzing system according to the present invention may be adapted so that, in order to construct the genetic map in the genetic map constructing section 42, the hybridization result is analyzed and processed by reading it from the array with the scanner 21 and the image information processing section 41, before specifying genetic markers. To this end, the image information processing section 41 is adapted to output information also to the genetic map constructing section 42, as shown in Fig. 12 (as indicated by arrow in the figure).

Information such as the genetic map constructed by the genetic map constructing section 42, information concerning the genetic markers specified by the genetic marker specifying section 43, the result of determination made by the genotype origin detecting section 44, the expression profile information generated by the expression profile information generating section 45, or hereditary factor information generated by the hereditary factor regulating section 46 can be temporarily stored in the memory 48. The memory 48 is provided in the analyzing section 40 as shown in Fig. 12, and serves as a storage section for storing various information used or generated in a gene interaction analyzing system according to the present invention. The storage operation of the memory 48 is controlled by the control section 47. The memory 48 is not limited to a particular structure, and may be realized, for example, by a semiconductor memory, such as RAM or ROM. Note that, the storage medium reading and writing section 23 described as an input section can be used as a storage section of the present invention. This will be described later in more detail in conjunction with the output section.

The analyzing section 40 of the structure shown in Fig. 12 includes the control section 47 for controlling the entire operation of the analyzing section 40, and in turn the entire operation of the gene interaction analyzing system. In the structure shown in Fig. 12, the control section 47 outputs control information to the image information processing section 41, the genetic map constructing section 42, the genetic marker specifying section 43, the spot marker information generating section 44, the expression profile information generating section 45, the hereditary factor regulating section 46, and the memory 48. These means operate based on the control information they receive, thereby operating the gene interaction analyzing system. It should be noted here that the control section 47 is also adapted to receive information from these means, and as such the flow of control information is indicate by the bidirectional arrow in Fig. 12.

### <Output section>

In the present invention, hereditary factors of the phenotype of interest are regulated by finding whether the spot marker information is linked to the expressed genes included in the expression profile information. To this end, a gene interaction analyzing system according to the present invention includes means, provided as an output section, for outputting a regulation result of hereditary factors.

The output section is not particularly limited, and at least one of, or preferably both of a display 26 for displaying a regulation result of hereditary factors on a display screen (soft copy), and a printer 25 for printing a regulation result of hereditary factors (hard copy) are provided. The display 26 is not limited to a particular structure, and various types of known displays such as a CRT, a liquid crystal display, and a plasma display can be used. The printer 25 is not limited to a particular structure, and known image forming devices such as an ink-jet printer and a laser printer can be used.

The output section is not just limited to the display 26 or printer 25, and other means can be used as well. For example, the external communications section 22 can be used as an output section. Specifically, the external communications section 22 allows for input and output of information to and from external devices by serving as both an input section and an output section. This enables the result of QTL analysis to be transmitted to other devices via external networks, etc, enabling a gene interaction analyzing system according to the present invention to be used more efficiently.

Specifically, when the gene interaction analyzing system is connected to external devices via LAN for example, the gene interaction analyzing system, installed in a research facility for example, can be shared with other researchers via information terminals such as personal computers. Further, the results of analysis obtained in the gene interaction analyzing system may be accumulated in an external server via a communications network, allowing the analysis result to be used more efficiently.

As the output section, the storage medium reading and writing section 23, described as an input section, can be suitably used. Specifically, in a gene interaction analyzing system according to the present invention, a drive for reading information from a storage medium can be used as an output section if the drive has a writing capability. The storage medium reading and writing section 23 is not limited to a particular structure, and known disk drives such as a hard disk drive, a flexible disk drive, a CD-ROM drive, and a DVD-ROM drive, or various memory cards or memory cartridges such as a USB memory can be suitably used for example, as described above in conjunction with the input section.

Note that, in the exemplary structure shown in Fig. 12, the system is realized by the analyzing section 40 and independently provided input and output sections, wherein the analyzing section 40 includes the image information processing section 41, the genetic map constructing section 42, the genetic marker specifying section 43, the spot marker information generating section 44, the expression profile information generating section 45, the hereditary factor regulating section 46, the control section 47, and the memory 48. However, the present invention is not just limited to this structure. For example, all means may be provided as a single unit, or some of the input sections and/or output sections may be integrated with the analyzing section 40. Further, the system may include means other than those shown in Fig. 12.

The analyzing section 40 is not just limited to a particular structure, and conventional arithmetic means, for example, such as a central processing unit (CDU) of a computer may be used. The operation of the analyzing section 40 is executed by a computer program.

### (III) Analyzing method by the gene interaction analyzing system

An analyzing method performed by a gene interaction analyzing system according to the present invention is not particularly limited. Specifically, the method may include 16 steps as represented in Fig. 13.

First, in step 301 (step will be denoted by "S" hereinafter), genetic map constructing information (names of chromosomes, genes, and genetic markers, and loci, etc.) is entered through input sections. In S302, the genetic map constructing section 42 constructs a genetic map based on the genetic map constructing information, and the genetic map is supplied to the genetic marker specifying section 43. In S303, the number of hybrid lines is entered through the input section. In S304, gene-presence-information (i.e., the result of DNA array analysis) of the hybrid individuals (targets individuals in Fig. 13) being analyzed is entered through the scanner 21 and the image information processing section 41 for each different hybrid line. In S305, genetic marker information is entered.

In S306, based on the entered gene-presence-information, genetic map, and genetic marker information, the genetic marker detecting section 43 determines a genetic marker that is present in each different hybrid line. Here, the result of determination of a genetic marker may be stored in the memory 48, or optionally displayed in the display 26. In S307, the genetic marker specified in S306 is compared with genetic markers immobilized on the DNA array, and spot marker information is generated from the individual spots of the DNA array. The spot marker information may be stored in the memory 48, or optionally displayed in the display 26. In S308, the presence or absence of an entry error is found (need for correction is determined). If there is an entry error (YES), the information is re-entered in S309 through, for example, the manual input section 24, and the sequence returns to S301. On the other hand, if correction is not required (NO), the sequence goes to S310.

In S310, a phenotypic value is entered through the input section. In S311, expression profile information of the hybrid individual being analyzed is entered through the input section. In S312, based on the expression profile information entered in S311, the expression profile information generating section 45 identifies genes with different expression levels. The identified genes may be stored in the memory 48, or optionally displayed in the display 26. In S313, a phenotype and gene of interest to be analyzed is entered through the input section.

In S314, interval mapping (QTL analysis) is performed based on the phenotypic value entered in S310 and the expressed genes identified in S312, so as to determine whether the plural pieces of spot marker information is linked to the phenotypic value and the expressed genes. In S315, based on the result of QTL analysis, associated genes and/or genetic markers are estimated, and the hereditary factors of the specified phenotype and gene are regulated based on the result of estimation. The regulation result of hereditary factor may be stored in the memory 48, or optionally displayed in the display 26. In S316, whether or not correction is required for the genes, etc. being analyzed is determined based on the result of analysis. If correction is required (YES), the sequence returns to S313. If correction is not required (NO), the sequence goes to S317. The result of analysis is outputted from the output section in S317. This completes the series of analysis procedures.

### (IV) Use of the present invention

Use of a gene interaction analyzing system according to the present invention is not particularly limited as long as interaction of more than one gene associated with a desired phenotype (trait) is analyzed in the species of interest.

As described earlier, a technique of expression profile analysis is known in which a group of genes are analyzed in clusters, or a network of gene expression is analyzed. These techniques are useful for the non-exclusive or comprehensive analysis of gene expression. However, since the techniques are for extracting closely associated genes with no other given information, they cannot be used for extracting hereditary factors closely associated with previously specified traits or genes to be analyzed. However, with a gene interaction analyzing system according to the present invention, a trait or gene of interest can be specified before actually analyzing gene interaction. That is, gene interaction can be analyzed differently from the expression profile analysis.

Thus, a gene interaction analyzing system according to the present invention is applicable not only to a gene interaction analyzing method but also to a gene search method, in which a search is made for genes associated with previously specified traits or genes to be analyzed.

In the expression profile analysis performed by the technique disclosed in Patent Document 6 as described in the BACKGROUND ART section, genes closely associated with a target evaluation index are extracted to estimate evaluation index data. The technique may appear to be similar to the present invention in the sense that the analysis is based on previously entered evaluation index data of interest. However, since the technique is for analyzing expression profiles based on the results obtained from a DNA array, it does not use the DNA array spots as genetic markers (spot marker information), nor does it perform a QTL analysis using such genetic markers.

The present invention does not analyze expression profiles, i.e., comprehensive analysis of gene expression is not performed. Rather, the analysis focuses on interaction between genes. In this way, a relationship between genes of interest can be established more clearly as compared with the comprehensive analysis. Therefore, the present invention can be suitably used for purposes requiring detailed analysis of genes which have already been analyzed by the comprehensive analysis.

Generally, in the cluster technique, a group of genes with different expression levels for a particular trait are detected in clusters. In order to find any relationship between these genes, each gene is annotated or a known pathway is analyzed, or, by an experimental biological approach, labeled genes are directly introduced into an organism. On the other hand, the present invention employs a genetic approach, whereby an analysis using a nucleic acid array is applied to a Mendelian segregation population, and interaction between individual genes contained in the cluster is identified in an exploratory manner. That is, interaction can be identified even for genes of unknown functions, and therefore any genetic association of a group of genes with a particular trait can be estimated. Since these genes are mapped, every gene can be introduced by variety improvement. Further, by the QTL analysis, the influence of the introduced genes on a trait can be statistically analyzed.

Specific use of the present invention is not particularly limited. For example, the gene interaction analyzing system can be used in a variety improvement method using genetic markers. As an example, traits or genes of interest may be specified for variety improvement, and genes or genetic markers closely associated with these traits or genes may be regulated. In this way, variety improvement can be performed more efficiently.

The type of organism to which an array of the present invention is applicable is not particularly limited, and any of plants, animals, and microorganisms may be used. Particularly, an array of the present invention can be used in the foregoing screening method in organisms that include chromosomes and obey the laws of Mendelian genetics. Examples of such an organism are, but not limited to, those commercially available and for which need for variety improvement is high.

In the case of plants, various crops (plant and farm products produced in agriculture, forestry, and fishery industries) can be used. Specific examples include: cereals such as rice, wheat, barley, rye, triticale, and corn; marine plants such as seaweed; various vegetables and flowers; and trees such as cedar or cypress. In the case of animals, various domestic animals can be used. Specific examples include: domestic mammals such as bovines, sheep, and pigs; domestic birds such as chickens and quails; fish such as yellowtail snapper, sea bream, carp, and sweetfish; insects such as honey bees, and silkworm; and shellfish such as oyster, ormer, and scallop. As microorganisms, bacteria such as *Escherichia coli*, yeasts, fungi, actinomycetes, and basidiomycetes can be used.

Among these examples, the cereals include crops such as rice, wheat, corn, and barley, which are cultivated worldwide and are strategically important. Thus, by using the present invention for the variety improvement of these plants, varieties with desirable traits can be efficiently produced.

The present invention can also be used for experimental animals and plants. Specific examples of experimental animals include mice, rats, *D. melanogaster*, and *C. elegans.* A specific example is *Arabidopsis thaliana.* Further, for the purpose of identifying genotypes with the present invention, the invention can be applied to humans.

As described above, in a gene interaction analyzing system according to the present invention, data of genetic markers for mapping is obtained together with gene expression data. With these data combined with phenotype data, the expression levels of respective genes are taken as target variables and are individually analyzed by a QTL analysis. That is, genes associated with traits or genes of interest are estimated after specifying traits or genes to be analyzed. It is therefore possible to efficiently estimate genes closely associated with traits or genes to be analyzed. Further, by interval mapping, hereditary factors can be estimated as quantitative trait loci (QTL) that exist between genetic markers, even when the genetic markers themselves are not sufficient to regulate the hereditary factors.

In sum, gene interaction can be efficiently analyzed under specific conditions, allowing for detailed analysis of gene interaction with the linkage information or QTL information, for example.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, in the present invention, the biosubstances or synthetic substances immobilized on an array are arranged based on the chromosomal order of genes corresponding to the biosubstances. The invention therefore has practical applications such as identification of a genotype, gene diagnosis, screening in variety improvement, or the like. Further, the invention improves reliability of an array analysis. Thus, the invention can be suitably used in the production of research reagents or samples using various types of arrays, or industries related to analytical techniques. Other applicable areas of the invention include crop production, animal production, and fisheries, in which identification of a genotype or variety improvement of organisms that follow the laws of Mendelian genetics is performed. The invention also has medical or pharmaceutical applications, such as gene diagnosis.

With the present invention, the genotype of each individual of a hybrid generation can be accurately determined or confirmed only by acquiring a genome of each individual of the hybrid generation and obtaining a hybridization result of a nucleic acid array. Thus, the invention can be suitably used in the production of research reagents or samples using various types of arrays, or industries related to analytical techniques. Other applicable areas of the invention include crop production, animal production, and fisheries, in which identification of a genotype or variety improvement of organisms that follow the laws of Mendelian genetics is performed. The invention also has medical or pharmaceutical applications, such as gene diagnosis.

Further, with the present invention, a QTL analysis can be efficiently performed with the array technique. Thus, the invention can be suitably used in the production of research reagents or samples using various types of arrays, or industries related to analytical techniques. Other applicable areas of the invention include crop production, animal production, and fisheries, in which identification of a genotype or variety improvement of organisms that follow the laws of Mendelian genetics is performed. The invention also has medical or pharmaceutical applications, such as gene diagnosis.

Further, with the present invention, gene interaction can be analyzed both efficiently and thoroughly under specific conditions. Thus, the invention can be suitably used in the production of research reagents or samples using various types of arrays, or industries related to analytical techniques. Other applicable areas of the invention include crop production, animal production, and fisheries, in which identification of a genotype or variety improvement of organisms that follow the laws of Mendelian genetics is performed. The invention also has medical or pharmaceutical applications, such as gene diagnosis.

## Claims

1. An array in which different kinds of biosubstances obtained from an organism of interest, or synthetic substances interacting with such biosubstances are arranged and immobilized on a support in an orderly manner,
the different kinds of biosubstances or the synthetic substances being arranged such that a chromosomal order of base sequence blocks corresponding to the biosubstances is ascertainable.

2. An array as set forth in claim 1, wherein at least part of the different kinds of biosubstances or the synthetic substances are arranged in the chromosomal order of the base sequence blocks corresponding to the biosubstances.

3. An array as set forth in claim 1 or 2, wherein the support includes a label that indicates the chromosomal order of the base sequence blocks corresponding to the biosubstances.

4. An array as set forth in claim 1,
wherein the biosubstances or the synthetic substances immobilized on the support each include sequence position information corresponding to the chromosomal order of the base sequence blocks corresponding to the biosubstances, and
wherein, in use, data is acquired and the sequence position information is read out, so as to rearrange sequences of the data in the chromosomal order.

5. An array as set forth in claim 1,
wherein the support comprises a collection of micro supports on which the biosubstances or the synthetic substances are individually immobilized,
wherein the micro supports each include sequence position information corresponding to the chromosomal order of the base sequence blocks corresponding to the biosubstances, and
wherein sequences of acquired data are rearranged in the chromosomal order based on the sequence position information.

6. An array as set forth in any one of claims 1 through 5, wherein the biosubstances comprise nucleic acids or polypeptides.

7. An array as set forth in claim 6, wherein the nucleic acid comprises DNA.

8. An array as set forth in claim 7, wherein the DNA comprises a genetic marker, genomic DNA, genomic DNA treated with a restriction enzyme, cDNA, EST, or synthetic oligoDNA.

9. An array as set forth in claim 7 or 8, wherein the DNA immobilized on the support is arranged based on a genetic map or physical map.

10. An array as set forth in any one of claims 7 through 9,
wherein genomic DNA treated with a restriction enzyme is used as target DNA.

11. An array as set forth in claim 10, wherein the target DNA is fractionated by size after the treatment with a restriction enzyme.

12. An array as set forth in any one of claims 1 through 5, wherein the polypeptides comprise proteins, fragments of protein, oroligopeptides.

13. An array as set forth in claim 12, wherein the proteins comprise enzymes, kinase, antibodies, receptors, or proteins with SH3 region.

14. An array as set forth in claim 12 or 13, wherein the proteins immobilized on the support are arranged based on a genetic map or physical map.

15. An array as set forth in any one of claims 1 through 14, wherein the support or micro support comprises an inorganic substrate, an organic film, or a bead.

16. An array as set forth in any one of claims 1 through 15, which comprises any one of a micro array, a macro array, a bead array, and a protein chip.

17. A producing process of an array, comprising the step of orderly arranging and immobilizing on a support different kinds of biosubstances obtained from an organism of interest, or synthetic substances interacting with such biosubstances,
said step comprising arranging and immobilizing the biosubstances or the synthetic substances according to the order in which genes corresponding to the biosubstances are coded for on a chromosome of the organism.

18. A producing process as set forth in claim 17, wherein the biosubstances comprise nucleic acids or polypeptides.

19. A genotype identification method, comprising identifying a target trait-including chromosome fragment, using the array of any one of claims 7 through 11, from hybrids obtained by crossing organisms.

20. An identification method as set forth in claim 19, wherein the organisms comprise laboratory animals and plants.

21. A gene diagnosis method for identifying human genotypes, using the identification method of claim 20.

22. A screening method for screening for a target trait-carrying variety from hybrids obtained by crossing organisms whose characteristics are to be improved, using the array of any one of claims 7 through 11.

23. A screening method as set forth in claim 22, wherein the living organisms crossed for variety improvement comprise laboratory animals and plants, domestic animals, or crops.

24. A screening method as set forth in claim 23, wherein the crops comprise cereals.

25. A screening method as set forth in claim 24, wherein the cereals comprise rice, wheat, corn, or barley.

26. A genotype analyzing and display system, comprising:
genotype origin detecting means for comparing (a) gene expression level information and polymorphism information comprehensively obtained through a hybridization analysis of hybrid individuals with the array of any one of claims 7 through 11 with (b) genetic information of parents of the hybrid individuals, and a genetic map of a species to which the hybrid individuals belong, so as to determine whether a genotype of a hybrid individual of interest derives from which parent; and
display information generating means for gathering a plurality of results obtained from the genotype origin detecting means and, based on the results, generating display information used to display a plurality of genotypes altogether on a chromosome basis, so as to determine whether individual genotypes derives from which parent.

27. A quantitative loci analyzing system which uses the array of any one of claims 7 through 11, and in which a genetic marker of a species of interest is immobilized on the array,
said quantitative loci analyzing system comprising:
genetic marker specifying means for comparing (a) comprehensive presence information of genes of hybrid individuals, obtained by hybridizing the array with a genomic sample obtained from the hybrid individuals of a certain hybrid line with (b) a genetic map of a species to which the hybrid individuals belong, and genetic marker information known in the species, so as to specify a genetic marker that exists in the hybrid line; and
quantitative loci detecting means for detecting a quantitative locus of a phenotype of interest of the hybrid individual, by confirming whether a phenotypic value indicative of the phenotype is linked to the genetic marker.

28. A gene interaction analyzing system which uses the array of any one of claims 7 through 11, and in which a genetic marker of a species of interest is immobilized on the array,
said gene interaction analyzing system comprising:
genetic marker specifying means for comparing (a) comprehensive presence information of genes of hybrid individuals, obtained by hybridizing the array with a genomic sample obtained from the hybrid individuals of a certain hybrid line (b) with a genetic map of species to which the hybrid individuals belong, and genetic marker information known in the species, so as to specify a genetic marker that exists in the hybrid line;
spot marker information generating means for comparing the specified genetic marker with the genetic marker immobilized on the support, so as to generate spot marker information, being genetic marker information for use in analysis, from hybridization results obtained from individual spots on the array; and
hereditary factor specifying means for specifying, with regard to an arbitrarily selected phenotype and gene to be analyzed, a hereditary factor of the selected phenotype by determining whether the phenotypic value indicative of the phenotype, and an expressed gene included in expression profile information obtained from the hybrid individual are linked to a plurality of spot marker information.

29. A genotype analyzing and display system, comprising:
genotype origin detecting means for comparing (a) gene expression level information and polymorphism information comprehensively obtained through a hybridization analysis of hybrid individuals using a nucleic acid array with (b) genetic information of parents of the hybrid individuals, and a genetic map of species to which the hybrid individuals belong, so as to determine whether a genotype of a hybrid individual of interest derives from which parent; and
display information generating means for gathering a plurality of results obtained from the genotype origin detecting means and, based on the results, generating display information used to display a plurality of genotypes altogether on a chromosome basis, so as to determine whether individual genotypes derives from which parent.

30. A genotype analyzing and display system as set forth in claim 29, wherein the nucleic acid array comprises a chromosomal location recognizable array in which a plurality of nucleic acid molecules immobilized thereon are arranged such that a chromosomal order of base sequence blocks corresponding to the nucleic acid molecules is ascertainable.

31. A genotype analyzing and display system as set forth in claim 29 or 30, further comprising genetic map constructing means for constructing, based on genetic map constructing information, a genetic map of a species to which the hybrid individuals belong.

32. A genotype analyzing and display system as set forth in claim 31, wherein the genetic map constructing information comprises names of genes and/or genetic markers known in the species, and chromosomal loci of the genes and/or genetic markers.

33. A genotype analyzing and display system as set forth in any one of claims 29 through 32, wherein the genotype origin detecting means determines a genotype as being homozygous for one of the parents, heterozygous, or unrecognizable to yield a result.

34. A genotype analyzing and display system as set forth in any one of claims 29 through 32, wherein the genotype origin detecting means uses genotype information and/or gene expression profile information of parents as genetic information of parents.

35. A genotype analyzing and display system as set forth in any one of claims 29 through 34, wherein the display information generating means generates display information including at least one of recombination number and recombination frequency of individual chromosomes.

36. A genotype analyzing and display system as set forth in any one of claims 29 through 35, wherein the display information generating means generates display information such that an origin of a genotype is identifiable based on different display colors or patterns.

37. A genotype analyzing and display system as set forth in any one of claims 29 through 36, comprising at least one of input means and output means.

38. A genotype analyzing and display system as set forth in claim 37, wherein the input means receives at least one of comprehensive expression level information of genes of the hybrid individuals, and genetic information of parents.

39. A genotype analyzing and display system as set forth in claim 38, wherein the input means receives genetic map constructing information.

40. A genotype analyzing and display system as set forth in any one of claims 37 through 39, comprising:
image reading means, provided as the input means, for enabling a hybridization result of the nucleic acid array to be read out as image information; and
image information processing means for analyzing an expression level of gene based on the image information and generating comprehensive expression level information of gene.

41. A genotype analyzing and display system as set forth in any one of claims 37 through 40, comprising manual input means, provided as the input means, for modifying at least one of: the comprehensive expression level information of gene of the hybrid individuals; the genetic information of parents; and the genetic map constructing information.

42. A genotype analyzing and display system as set forth in any one of claims 37 through 41, wherein the output means comprises at least one of: image display means for displaying the display information on a screen; and printing means for printing the display information.

43. A genotype analyzing and display system as set forth in any one of claims 37 through 41, wherein the input means and the output means comprise external information input-output means for sending and receiving information to and from an external device.

44. A genotype analyzing and display system as set forth in any one of claims 29 through 43, wherein the nucleic acid array comprises a DNA array on which DNA is immobilized.

45. A genotype analyzing and display system as set forth in claim 44, wherein the DNA immobilized on the DNA array comprises a genetic marker, genomic DNA, genomic DNA treated with a restriction enzyme, cDNA, EST, or synthetic oligoDNA.

46. A genotype analyzing and display system as set forth in any one of claims 29 through 45, which comprises any one of a micro array, a macro array, and a bead array.

47. A genotype identification method, comprising identifying a target trait-including chromosome fragment, using the genotype analyzing and display system of any one of claims 29 through 46, from hybrids obtained by crossing organisms.

48. An identification method as set forth in claim 47, wherein the organisms comprise laboratory animals and plants.

49. A screening method for screening for a target trait-carrying variety from hybrids obtained by crossing organisms whose characteristics are to be improved, using the genotype analyzing and display system of any one of claims 29 through 46.

50. A screening method as set forth in claim 49, wherein the organisms crossed for variety improvement comprise laboratory animals and plants, domestic animals, or crops.

51. A quantitative loci analyzing system, comprising:
genetic marker specifying means for comparing (a) comprehensive presence information of genes of hybrid individuals, obtained by hybridizing a genomic sample of the hybrid individuals of a certain hybrid line with a nucleic acid array on which a genetic marker of a species of interest is immobilized with (b) a genetic map of a species to which the hybrid individuals belong, and genetic marker information known in the species, so as to specify a genetic marker that exists in the hybrid line; and
quantitative loci detecting means for detecting a quantitative locus of a phenotype of interest of the hybrid individual, by confirming whether a phenotypic value indicative of the phenotype is linked to the genetic marker.

52. A quantitative loci analyzing system as set forth in claim 51, wherein the nucleic acid array comprises a chromosomal location recognizable array in which a plurality of nucleic acid molecules immobilized thereon are arranged such that a chromosomal order of base sequence blocks corresponding to the nucleic acid molecules is ascertainable.

53. A quantitative loci analyzing system as set forth in claim 51 or 52, further comprising genetic map constructing means for constructing, based on genetic map constructing information, a genetic map of a species to which the hybrid individuals belong.

54. A quantitative loci analyzing system as set forth in claim 53, wherein the genetic map constructing information comprises names of genes and/or genetic markers known in the species, and chromosomal loci of the genes and/or genetic markers.

55. A quantitative loci analyzing system as set forth in any one of claims 51 through 54, wherein the genetic marker information used by the genetic marker specifying means comprises a genetic marker with polymorphism.

56. A quantitative loci analyzing system as set forth in claim 55, wherein the genetic marker comprises SNP or RFLP.

57. A quantitative loci analyzing system as set forth in any one of claims 51 through 56, wherein the quantitative loci detecting means detects a quantitative locus of phenotype by interval mapping.

58. A quantitative loci analyzing system as set forth in any one of claims 51 through 57, comprising:
image reading means for enabling a hybridization result of the nucleic acid array to be read out as image information; and
image information processing means for analyzing the image information and generating comprehensive expression level information of gene.

59. A quantitative loci analyzing system as set forth in any one of claims 51 through 57, comprising at least one of input means and output means.

60. A quantitative loci analyzing system as set forth in claim 59, wherein the input means receives at least one of the genetic marker information and the phenotypic value.

61. A quantitative loci analyzing system as set forth in claim 60, wherein the input means receives at least one of the genetic map and the genetic map constructing information.

62. A quantitative loci analyzing system as set forth in any one of claims 59 through 61, comprising manual input means, provided as the input means, for modifying at least one of: the comprehensive presence information of gene of the hybrid individuals; the genetic marker information, and the genetic map constructing information.

63. A quantitative loci analyzing system as set forth in any one of claims 59 through 62, wherein the output means comprises at least one of image display means for displaying an analysis result on a screen; and printing means for printing an analysis result.

64. A quantitative loci analyzing system as set forth in any one of claims 59 through 63, wherein the input means and the output means comprise external information input-output means for sending and receiving information to and from an external device.

65. A quantitative loci analyzing system as set forth in any one of claims 51 through 64, wherein the nucleic acid array comprises a DNA array on which DNA is immobilized.

66. A quantitative loci analyzing system as set forth in any one of claims 51 through 65, wherein the nucleic acid array comprises a micro array, a macro array, or a bead array.

67. A quantitative trait analyzing method for analyzing a quantitative trait of an organism, using the quantitative loci analyzing system of any one of claims 51 through 66.

68. A gene searching method for searching for a gene associated with expression of a trait of interest, using the quantitative loci analyzing system of any one of claims 51 through 66.

69. A variety improvement method for organisms, which uses the quantitative loci analyzing system of any one of claims 51 through 66.

70. A variety improvement method as set forth in claim 69, wherein the organisms comprise laboratory animals and plants, domestic animals, or crops.

71. A gene interaction analyzing system, comprising:
genetic marker specifying means for comparing (a) comprehensive presence information of genes of hybrid individuals, obtained by hybridizing a genomic sample of the hybrid individuals of a certain hybrid line with a nucleic acid array on which a genetic marker of a species of interest is immobilized with (b) a genetic map of a species to which the hybrid individuals belong, and genetic marker information known in the species, so as to specify a genetic marker that exists in the hybrid line;
spot marker information generating means for comparing the specified genetic marker with the genetic marker immobilized on the nucleic acid array, so as to generate spot marker information, being genetic marker information for use in analysis, from hybridization results obtained from individual spots on the nucleic acid array; and
hereditary factor specifying means for specifying, with regard to an arbitrarily selected phenotype and gene to be analyzed, a hereditary factor of the selected phenotype by determining whether the phenotypic value indicative of the phenotype, and an expressed gene included in expression profile information obtained from the hybrid individual are linked to a plurality of spot marker information.

72. A gene interaction analyzing system as set forth in claim 70, wherein the nucleic acid array comprises a chromosomal location recognizable array in which a plurality of nucleic acid molecules immobilized thereon are arranged such that a chromosomal order of base sequence blocks corresponding to the nucleic acid molecules is ascertainable.

73. A gene interaction analyzing system as set forth in claim 71 or 72, further comprising genetic map constructing means for constructing, based on genetic map constructing information, a genetic map of a species to which the hybrid individuals belong.

74. A gene interaction analyzing system as set forth in claim 73, wherein the genetic map constructing information comprises names of genes and/or genetic markers known in the species, and chromosomal loci of the genes and/or genetic markers.

75. A gene interaction analyzing system as set forth in any one of claims 71 through 74, wherein the genetic marker information used by the genetic marker specifying means comprises a genetic marker with polymorphism.

76. A gene interaction analyzing system as set forth in claim 75, wherein the genetic marker comprises SNP or RFLP.

77. A gene interaction analyzing system as set forth in any one of claims 71 through 76, wherein the spot marker information generating means generates spot marker information only for a genetic marker spot found by hybridization.

78. A gene interaction analyzing system as set forth in claim 77, wherein the spot marker information generating means generates spot marker information by including position information of a genetic marker immobilized on the nucleic acid array.

79. A gene interaction analyzing system as set forth in any one of claims 71 through 78, comprising expression profile information generating means for analyzing an expression profile in regard to a comprehensive gene expression level obtained from the hybrid individual, so as to generate expression profile information of the hybrid individual.

80. A gene interaction analyzing system as set forth in claim 79, wherein the expression profile information generating means generates expression profile information of the hybrid individual by comprehensively measuring gene expression, using at least one of a micro array, a macro array, a bead array, and a differential display.

81. A gene interaction analyzing system as set forth in claim 80, wherein the expression profile information generating means generates expression profile information using a nucleic acid array used to obtain comprehensive presence information of gene of the hybrid individual, or a nucleic acid array on which the same sample has been spotted.

82. A gene interaction analyzing system as set forth in any one of claims 71 through 81, wherein the nucleic acid array comprises a DNA array on which DNA is immobilized.

83. A gene interaction analyzing system as set forth in any one of claims 71 through 82, wherein the nucleic acid array comprises a micro array, a macro array, or a bead array.

84. A gene interaction analyzing system as set forth in any one of claims 71 through 83, wherein the hereditary factor specifying means specifies a hereditary factor of a phenotype based on a quantitative trait locus (QTL) that exists among genetic markers obtained by interval mapping.

85. A gene interaction analyzing system as set forth in claim 84, wherein the hereditary factor specifying means uses information of expression level of a gene associated with the genetic marker, so as to specify a hereditary factor of the phenotype.

86. A gene interaction analyzing system as set forth in any one of claims 71 through 85, comprising at least one of input means and output means.

87. A gene interaction analyzing system as set forth in claim 86, wherein the input means receives at least one of: comprehensive presence information of gene of the hybrid individual; the genetic marker information; the phenotypic value; and the expression profile information.

88. A gene interaction analyzing system as set forth in claim 87, wherein the input means receives at least one of the genetic map and the genetic map constructing information.

89. A gene interaction analyzing system as set forth in any one of claims 86 through 88, comprising:
image reading means, provided as the input means, for enabling a hybridization result of the nucleic acid array to be read out as image information; and
image information processing means for analyzing an expression level of gene based on the image information and generating comprehensive expression level information of gene.

90. A gene interaction analyzing system as set forth in claim 89, wherein the input means receiving the expression profile information comprises image information reading means.

91. A gene interaction analyzing system as set forth in any one of claims 86 through 90, comprising manual input means, provided as the input means, for modifying at least one of: the comprehensive presence information of gene of the hybrid individuals; the genetic marker information, and the genetic map constructing information.

92. A gene interaction analyzing system as set forth in any one of claims 86 through 91, wherein the output means comprises at least one of image display means for displaying an analysis result on a screen; and printing means for printing an analysis result.

93. A gene interaction analyzing system as set forth in any one of claims 86 through 92, wherein the input means and the output means comprise external information input-output means for sending and receiving information to and from an external device.

94. A gene interaction analyzing method for analyzing interaction between genes, using the gene interaction analyzing system of any one of claims 71 through 93.

95. A gene searching method for searching for a gene associated with a trait of interest, using the gene interaction analyzing system of any one of claims 71 through 93.

96. A variety improvement method for organisms, which uses the gene interaction analyzing system of any one of claims 71 through 93.

97. A variety improvement method as set forth in claim 96, wherein the organisms comprise laboratory animals and plants, domestic animals, or crops.
